# EUROPEAN PATENT SPECIFICATION

(11) **EP 1 858 444 B1**
(45) Date of publication and mention of the grant of the patent: **28.08.2013**
(21) Application number: 06738301.8
(22) Date of filing: 14.03.2006
(51) Int. Cl.: A61F 2/06

(54) **SEGMENTED ENDOPROSTHESIS**
SEGMENTIERTE ENDOPROTHESE
ENDOPROTHESE SEGMENTEE

(30) Priority: 14.03.2005 US 661542 P; 13.03.2006 US 374923; 13.03.2006 US 375380; 13.03.2006 US 375381
(43) Date of publication of application: 28.11.2007
(73) Proprietor: ABBOTT LABORATORIES, Abbott Park IL 60064-3500 (US)
(72) Inventor: NEWHAUSER, Richard, San Francisco, California 94109 (US); YRIBARREN, Travis, R., San Mateo, California 94401 (US)
(74) Representative: McLeish, Nicholas Alistair Maxwell
(86) International application number: PCT/US2006/009226
(87) International publication number: WO 2006/099449

(56) References cited:
- WO-A-00/15151
- WO-A-02/24111
- WO-A-03/075797
- WO-A-2004/043221
- US-A1- 2002 007 211
- US-A1- 2003 078 649
- US-B1- 6 565 599

## Description

### 1. The Field of the Invention

The present invention relates to an endoprosthesis deliverable and deployable within a body vessel of a human or animal. More particularly, the invention relates to a segmented endoprosthesis with improved functionality.

### 2. The Relevant Technology

Stents, grafts, and a variety of other endoprostheses are well known and used in interventional procedures, such as for treating aneurysms, for lining or repairing vessel walls, for filtering or controlling fluid flow, and for expanding or scaffolding occluded or collapsed vessels. Such endoprostheses can be delivered and used in virtually any accessible body lumen of a human or animal, and can be deployed by any of a variety of recognized means. One recognized indication of an endoprosthesis, such as a stent, is for the treatment of atherosclerotic stenosis in blood vessels. For example, after a patient undergoes a percutaneous transluminal coronary angioplasty or similar interventional procedure a stent is often deployed at the treatment site to improve the results of the medical procedure and reduce the likelihood of restenosis. The stent is configured to scaffold or support the treated blood vessel; if desired, it can also be loaded with a beneficial agent so as to act as a delivery platform to reduce restenosis or the like.

An endoprosthesis, such as a stent, is typically delivered by a catheter delivery system to a desired location or deployment site inside a body lumen of a vessel or other tubular organ. The intended deployment site may be difficult to access by a physician and often involves traversing the delivery system through a tortuous luminal pathway. Thus, it can be desirable to provide the endoprosthesis with a sufficient degree of flexibility during delivery to allow advancement through the anatomy to the deployment site. Moreover, it may be desirable for the endoprosthesis to retain structural integrity while flexing and bending during delivery so that cracks do not form.

Current stent design, typically are composed of a series of repeated rings that are connected in series. The stent in a Superficial Femoral Artery (SFA) application undergoes longitudinal, bending, torsional, tensional and radial cyclical loading that can lead to fatigue failures. The stent connection sections or connection elements that join the rings also transmit stress from ring to ring under longitudinal, bending, or torsional loading. In addition, when the stent goes around a curve the connecting elements or sections require the portions of the ring apposed to the outside of the curve to lengthen and the portions of the ring apposed to the inside of the curve to shorten. Lengthening and shortening portions of the ring increase the maximum stress because the ring cannot expand evenly promoting fatigue failures. Current endoprosthesis designs which are subjected to these forces generally fail. Failure can result in crack formation and possible stent fracture. In the event of stent fracture, the sharp edges may puncture the vessel, muscle tissue or cause bleeding. Additionally, the fractured stent may cause thrombus formation or blockage within the vessel.

In WO 2004/043221 there is described a family of stents where the relative hardness/softness of regions of the stent can differ from other regions of the stent to provide additional patient comfort and resistance to radial forces. In one example there is provided a family of stents with interstice configurations that are said to facilitate flexibility, durability and/or proper installation.

Although various endoprostheses have been developed to address one or more of the aforementioned performance characteristics, there remains a need for a more versatile design that improves one or more performance characteristics without sacrificing the remaining characteristics.

Therefore, it would be advantageous to have an endoprosthesis configured to have improved flexibility after deployment where the connection section absorbed or did not transmit the longitudinal, torsional, or bending loading to the stent rings or segment. Also, it would be beneficial to have an endoprosthesis configured to allow for adjacent annular elements to move relative to each other to enhance delivery in tortuous luminal pathways.

### SUMMARY OF THE INVENTION

The present invention relates to a segmented endoprosthesis for delivery into a lumen of a body. The endoprostheses is configured to include a plurality of longitudinally-adjacent annular elements that can move independently from each other. The segmented endoprosthesis provides improved deliverability, strength, flexibility, and/or functionality during and after deployment. The use of a segmented endoprosthesis combines the characteristics of multiple small endoprostheses into a standard- or regular-sized endoprosthesis.

According to the present invention there is provided a segmented endoprosthesis comprising: a first annular element; a second annular element adjacent to the first annular element; a shock absorbing inter-connector coupled to the first annular element and second annular element, the shock absorbing inter-connector being comprised of at least two bending members and each of the at least two bending members being comprised of at least one bending point; a first coupling that couples the shock absorbing inter-connector to the first annular element; and a second coupling that couples the shock absorbing inter-connector to the second annular element, wherein the first coupling couples a first end of each of the at least two bending members to the first annular element, and the second coupling couples a second end of each of the at least two bending members to the second annular element such that the shock absorbing inter-connector reduces the forces transferred from the first annual element to the second annular element during placement within a body lumen.

The inter-connector can reduce stress and forces propagating between adjacent positions of the endoprosthesis. This inter-connector can flex so that the annular elements can move relative to one another and facilitate delivery to and placement within a body lumen. Further, this inter-connector can enable the endoprosthesis to flex during movement of the body lumen following deployment.

In another configuration, the segmented endoprosthesis includes a first annular element flexibly-resiliently coupled to a second annular element by first and second flexibly-resilient inter-connectors. The first annular element has a first end that includes both a first strut element and a second strut element, while the second annular element has a second end that is adjacent to the first end of the first annular element. Adjacent to the third strut element is the first stent element and the second strut element is adjacent to the fourth strut element. The first flexibly-resilient inter-connector is coupled to the first strut element and the third strut element, and the second flexibly-resilient inter-connector is coupled to the second strut element and the fourth strut element. Additionally, the first and second inter-connectors are positioned on the endoprosthesis so that when the endoprosthesis bends in a first direction the first inter-connector collapses and the second inter-connector expands, or vice versa.

In another configuration, a flexible, segmented endoprosthesis incldues a first annular element adjacent to a second annular element. A plurality of shock absorbing members or structures each having a first end and a second end are disposed between the first and second annular elements. The first end is coupled to the first annular element and the second end is coupled to the second annular element.

There is also described a decouplable segmented that includes a first annular element having a plurality of first decouplable inter-connectors at a first end and a second annular element having a plurality of second decouplable inter-connectors at a second end. At least one of the plurality of second decouplable inter-connectors can be releasably coupled with at least one of the plurality of first decouplable inter-connectors when the segmented endoprosthesis is in a delivery orientation. Additionally, when in the delivery configuration the decouplable inter-connectors can be configured to transmit axial loads while preventing one segment to move independent of another.

In one configuration, the decouplable segmented endoprosthesis can include a plurality of sub-endoprostheses releasably coupled together when the segmented endoprosthesis is in a delivery orientation. Each of the plurality of sub-endoprostheses can decouple from adjacent mini-endoprostheses when the endoprosthesis is expanded into a deployed orientation. Accordingly, the endoprosthesis can include a first sub-endoprosthesis that can include at least a first decouplable inter-connector and a second sub-endoprosthesis that can include at least a second decouplable inter-connector that interlocks with the at least a first decouplable inter-connector when the endoprosthesis is in the delivery orientation.

In another configuration, a sub-endoprosthesis for use in the segmented endoprosthesis can include a first annular element that can have a lumen extending from a first end to a second end. A first plurality of first decouplable inter-connectors can be disposed on the first end of the annular element and a second plurality of second decouplable inter-connectors can be disposed on the second end of the annular element. At least one of the second plurality of second decouplable inter-connectors can be configured to releasably couple with at least one of the first plurality of first decouplable inter-connectors on a second annular element that is substantially the same as the first annular element.

There is also described a segmented endoprosthesis that includes a series of longitudinally-adjacent sub-endoprostheses in which the segmented sub-endoprosthesis include a first annular element that has a plurality of first bumpers at a first end and a second annular element that has a plurality of second bumpers at a second end. At least one of the plurality of second bumpers can abut with at least one of the plurality of first bumpers when the segmented endoprosthesis is in a delivery orientation.

In one configuration, the series of sub-endoprostheses can have bumpers that separate each other and form a segmented endoprosthesis. Such a segmented endoprosthesis can include a plurality of sub-endoprostheses. Each sub-endoprosthesis can be in contact with longitudinally-adjacent sub-endoprostheses when the segmented endoprosthesis is in a delivery orientation. Also, each of the plurality of sub-endoprostheses can separate from the longitudinally-adjacent sub-endoprostheses when the endoprosthesis is expanded into a deployed orientation. The segmented endoprosthesis can include a first sub-endoprosthesis that can include at least a first bumper, and a second sub-endoprosthesis that can include at least a second bumper that abuts and contacts the first bumper when the endoprosthesis is in the delivery orientation.

In another configuration, a sub-endoprosthesis for use with an endoprosthesis can have a bumper. The sub-endoprosthesis can include a first annular element that can have a lumen extending from a first end to a second end. A first plurality of first bumpers can be disposed on the first end of the annular element and a second plurality of second bumpers can be disposed on the second end of the annular element.

These and other embodiments and features of the present invention will become more fully apparent from the following description, drawings, and/or appended claims, or may be learned by the practice of the invention as set forth hereinafter.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only typical embodiments of the invention and are therefore not to be considered limiting of its scope. The invention will be described and explained with additional specificity and detail through the use of the accompanying drawings, in which:
Figure 1 is a planar side view of a portion of an embodiment of an exemplary endoprosthesis in accordance with the present invention;
Figure 2A is a side view illustrating a portion of an embodiment of an endoprosthesis in a deployed orientation;
Figure 2B is a side view illustrating a portion of an embodiment of an endoprosthesis in a delivery orientation;
Figure 3A is a perspective view of an annular element: in a delivery orientation;
Figure 3B is a perspective view of a tubular endoprosthesis in a delivery orientation and having the annular element of Figure 3A;
Figure 3C is a perspective view of the annular element of Figure 3A in a deployed orientation;
Figure 3D is a perspective view of a series of annular elements in accordance with Figure 3A, wherein the series of annular elements are in a deployed orientation;
Figure 3E is a perspective view of a tubular endoprosthesis in a deployed orientation and having a series of annular elements in accordance with Figure 3A;
Figure 4A is a perspective view of an annular element in a delivery orientation;
Figure 4B is a perspective view of a series of annular elements in a deployed orientation, the series of annular elements are in accordance with Figure 4A;
Figure 4C is a perspective view of a tubular endoprosthesis in a delivery orientation and having a series of annular elements in accordance with Figure 4A;
Figures 5A-5D are side views of decouplable inter-connecters;
Figures 6A-6D are side views of annular element bumpers;
Figure 7A is a side view illustrating a portion of another endoprosthesis in a collapsed or delivery orientation;
Figure 7B is a side view illustrating a portion of the endoprosthesis of Figure 7A in a collapsed orientation;
Figure 7C-D are perspective views of the endoprosthesis of Figure 7A in a tubular, delivery orientation, prior to expansion, and having a series of annular elements;
Figure 7E-F are side views of the endoprosthesis of Figure 7A in an expanded or delivered orientation;
Figures 7G-H are perspective views of the endoprosthesis of Figure 7A in a tubular, expanded orientation;
Figure 8A is a side view illustrating a portion of another endoprosthesis in a collapsed orientation;
Figure 8B is a side view illustrating a portion of the endoprosthesis of Figure 8A in an expanded or delivered orientation;
Figure 8C is a perspective view of the endoprosthesis of Figure 8A in a tubular, expanded or delivered orientation, and having a series of annular elements; and
Figures 9A-B are side views of another endoprosthesis, the endoprosthesis having a coiled and offset configuration.

### DETAILED DESCRIPTION OF THE PREFERRED EMBODIMENTS

The present invention includes various embodiments of endoprostheses for delivery into a lumen of a body. The endoprostheses is configured to have improved functionality by being segmented so that adjacent annular elements can move independently. As such, the segmented endoprosthesis has improved delivery and placement within a tortuous luminal pathway.

The use of a segmented endoprosthesis that is sectioned into multiple annular elements or multiple sub-endoprosthesis can enable delivery around tight corners. For example, one annular element or sub-endoprosthesis can be pushed through a tight corner in such a manner that a flexible inter-connection allows each annular element or sub-endoprosthesis to move independently. In another example, the inter-connection can allow the adjacent annular elements to separate from each other in order to avoid generating undue stress at a corner, and then rejoin at a straight section. In still another example, the interconnection can be prepared in such a manner that the different annular elements or sub-endoprosthesis are selectively held together by contact. As such, the inter-connection is not unduly stressed and is less susceptible to cracking. Thus, the segmented endoprostheses having the multiple annular elements or sub-endoprosthesis increase the ability for easy deployment and retain substantial structural integrity of the endoprosthesis after deployment.

### I. Segmented Endoprosthesis

In accordance with the present invention, a segmented endoprosthesis is provided for improved delivery within a body lumen of a human or other animal. Examples of segmented endoprostheses can include stents, filters, grafts, valves, occlusive devices, trocars, aneurysm treatment devices, or the like. Additionally, a segmented endoprosthesis can be configured for a variety of intralumenal applications, including vascular, coronary, biliary, esophageal, urological, gastrointestinal, or the like. The segmented endoprostheses can be prepared from multiple annular elements or sub-endoprosthesis that are interconnected by flexible, degradable, bumper, and/or decouplable inter-connectors. As such, the inter-connectors inhibit stresses or strains from being transmitted between adjacent annular elements or sub-endoprosthesis. Also, the inter-connectors inhibit excessive or destructive stresses or strains from being generated at the junctions between the adjacent annular elements or sub-endoprosthesis. Thus, adjacent annular elements or sub-endoprosthesis can be separated by junctions that inhibit crack formation and failure of the endoprosthesis.

Generally, an endoprosthesis of the present invention includes at least a first set of interconnected strut elements that cooperatively define an annular element or sub-endoprosthesis. A strut element can be more generally described as an endoprosthetic element, wherein all well-known endoprosthetic elements can be referred to here as a "strut element" for simplicity. Usually, each strut element can be defined by a cross-sectional profile as having a width and a thickness, and including a first end and a second end bounding a length. The stent element can be substantially linear, arced, rounded, squared, combinations thereof, or other configurations. The strut element can include a bumper, crossbar, connector, inter-connector, intersection, elbow, foot, ankle, toe, heel, medial segment, lateral segment, combinations thereof, or the like, as described in more detail below. The strut element can have improved structural integrity by including crack-inhibiting features, which are described in detail in the incorporated references.

Usually, the annular elements or sub-endoprosthesis can include a plurality of circumferentially-adjacent crossbars that are interconnected end-to-end by an elbow connection, intersection, or a foot extension. As such, at least one annular element or sub-endoprosthesis can include an elbow, intersection, or a foot extension ("foot") extending between at least one pair of circumferentially-adjacent crossbars. The elbow or foot can thus define an apex between the pair of circumferentially-adjacent crossbars of the annular element or sub-endoprosthesis. Also, an intersection can have a shape similar to a cross so as to provide a junction between two coupled pairs of circumferentially-adjacent crossbars.

The elbow can be configured in any shape that connects adjacent ends of circumferentially-adjacent crossbars, and can be described as having a U-shape, V-shape, L-shape, or the like. An intersection can be configured in any shape that connects longitudinal and circumferentially adjacent crossbars, and can be described as having a cross shape, X-shape, H-shape, K-shape, or the like. The foot can have a foot shape having a first foot portion extending circumferentially from an end of one of the adjacent strut members and a second foot portion extending circumferentially from a corresponding end of the other of the circumferentially-adjacent strut members. In combination, the first and second foot portions generally define an ankle portion connected to a toe portion through a medial segment and the toe portion connected to a heel portion through a lateral segment.

As described herein, an endoprosthesis, in one configuration, includes two or more interconnected annular elements or sub-endoprosthesis. Each annular element or sub-endoprosthesis generally defines a ring-like structure extending circumferentially about a longitudinal or central axis. The cross-sectional profile of each annular element or sub-endoprosthesis can be at least arcuate, circular, helical, or spiral, although alternative cross-sectional profiles, such as oval, oblong, rectilinear or the like, can be used.

When the endoprosthesis includes multiple spaced apart annular elements or sub-endoprostheses, a first annular elements can be aligned longitudinally adjacent to a second annular element along the longitudinal axis. The first and second annular elements can be interconnected by flexible, degradable, bumper, and/or decouplable inter-connectors. The inter-connectors can be considered as strut elements for the purposes of the invention. As such, the inter-connectors can be strut elements that interconnect adjacent annular elements or sub-endoprostheses so as to improve the structural integrity of the endoprosthesis by inhibiting the buildup of stresses or strains at the interconnection or inhibiting propagation of stresses or strains between adjacent annular elements or sub-endoprostheses. The first and second annular elements or sub-endoprostheses generally define a tubular structure. For example, each annular element or sub-endoprosthesis can define a continuous closed ring such that the longitudinally-aligned annular elements or sub-endoprostheses form a closed tubular structure having a central longitudinal axis. Alternatively, each annular element or sub-endoprosthesis can define an open ring shape such that a rolled sheet, open tubular, or "C-shape" type structure is defined by the annular elements. That is, the annular element or sub-endoprosthesis is not required to be closed. Furthermore, each annular element or sub-endoprosthesis can define substantially a 360-degree turn of a helical pattern or spiral, such that the end of one annular element or sub-endoprosthesis can be joined with the corresponding end of a longitudinally-adjacent annular element or sub-endoprosthesis to define a continuous helical pattern along the length of the endoprosthesis.

### A. Shock Resistant/Absorbing Endoprosthesis

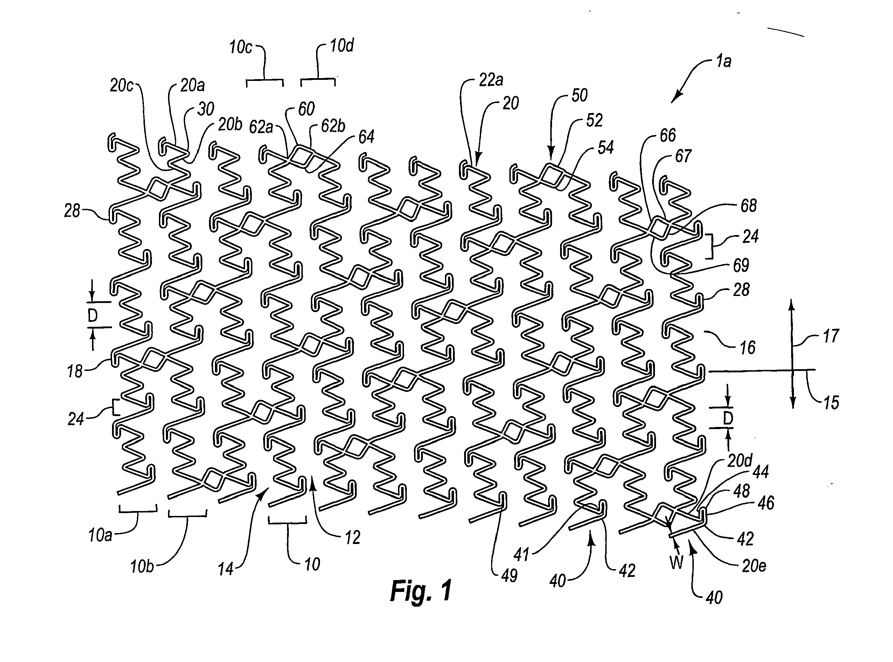

One configuration of the present invention include an endoprosthesis configured to flex during deployment and after being set. Figures 1-2B illustrate embodiments of endoprostheses that include a plurality of annular elements that are interconnected by shock resistant or absorbing member or shock absorbers. The shock absorbers, or means for reducing force transmission between adjacent annular elements, allow the individual annular elements to flex, move longitudinally, and/or bend with respect to each other while in a collapsed or deployed configuration. Additionally, the shock absorbers allow the individual annular elements to flex radially, circumferentially, axially, and longitudinally while deployed.

Figure 1 is a side view of a flattened portion of an embodiment of an endoprosthesis 1a. The illustrated endoprosthesis is a stent, but it will be understood that the benefits and features of the present invention are also applicable to other types of endoprosthesis or other medical devices known to those skilled in the art.

For purposes of clarity and not limitation, the endoprosthesis 1a is illustrated in a planar format. As shown, the endoprosthesis 1a includes a plurality of annular elements 10 aligned longitudinally adjacent to each other along a longitudinal axis 15. Although only two interconnected annular elements need to be provided for the endoprosthesis, it is possible that an endoprosthesis include more than two annular elements 10. As depicted in Figure 1, least a first annular element 10a and a second annular element 10b are identified.

Each annular element 10 includes a set of interconnected strut elements, shown as strut crossbars 20, which are disposed circumferentially about the longitudinal axis 15; the circumferential direction is represented by arrow 17. Each crossbar 20 has a first end 22a and a second end 22b, referenced generally as end 22. The first end 22a of selected circumferentially-adjacent crossbars 20a-b are interconnected at elbows 30 that are proximate to a first longitudinal side 12 of each annular element 10, and the second end 22b of selected circumferentially-adjacent crossbars 20b-c are interconnected to define elbows 30 that are proximate to a second longitudinal side 14 of the annular element.

Each annular element 10 can be expanded to a deployed configuration as shown in Figure 1 by altering or opening the angle of the elbows 30 interconnecting the circumferentially-adjacent crossbars 20, or can be collapsed into a deployable configuration by closing the angle of the elbows 30. Also, circumferentially-adjacent elbows 30 on each side 12, 14 of the annular element 10 can be spaced apart by a circumferential distance D, such that each annular element 10 is expanded by increasing the distance D and collapsed by decreasing the distance D. At any given condition between the delivery configuration and the deployed configuration, the distance D can be balanced or constant from one set of circumferentially-adjacent elbows to the next, or it can be varied if desired.

Selected elbows 30 on each side 12, 14 of the annular element 10 are defined by interconnecting corresponding ends 22 of circumferentially-adjacent crossbars 20a-b directly together to form a zigzag pattern of alternating U-shapes, V-shapes, L-shapes, combinations thereof, or the like when deployed. Alternatively, an elbow 30 can be provided between the corresponding ends of adjacent crossbars to form another contoured shape, such as by using a straight elbow member to form a flat connection configuration.

Figure 1 also depicts an embodiment of a foot extension 40 that extends between a pair 24 of circumferentially-adjacent crossbars 20d-e of each annular element 10. As depicted, the foot extension 40 includes an ankle 41 that circumferential ly couples an end 22 of one of the adjacent crossbars 20d to a medial segment 44. The medial segment 44 extends from the ankle 41 to a toe 48 that circumferentially couples the medial segment to a lateral segment 46. The lateral segment 46 extends from the toe 48 to a heel 42 that circumferentially couples the lateral segment to the next circumferentially-adjacent crossbar 20e. Accordingly, the juncture of the crossbar 20d and the medial segment 44 defines a circumferentially-extending toe portion 48 of the foot extension 40; the juncture of the medial segment 44 and the lateral segment 46 defines a circumferentially-extending toe portion 48 of the foot extension 40; and the juncture of the lateral segment 46 and crossbar 20e defines a circumferentially-extending toe portion 48 of the foot extension 40. Each portion of the foot extension 40, as well as each of the circumferentially-adjacent crossbars 20, has a substantially uniform cross-sectional profile illustrated by a substantially uniform width W and thickness (not shown).

For purposes of discussion and not limitation, Figure 1 shows that a toe portion 48 can extend in a first circumferential direction a distance greater than the distance the heel portion 42 of the foot extension 40 extends in an opposite circumferential direction. As such, the entirety of the foot extension 40 can extend in the circumferential direction of the toe portion 48. Furthermore, at least one of the medial segment 44 or lateral segment 46 can open foot region 49.

The adjacent annular elements 10a-10b or 10c-10 are interconnected with an inter-connector 50, having the form of a means for reducing force transmission between adjacent annular elements. Stated another way, the inter-connector 50, optionally referred to as a shock absorber or shock absorbing connector, includes one or more shock absorbing members that allow limited movement of adjacent annular elements, while reducing the possibility of cracking and fatigue failure due to the movement of adjacent annular elements. As such, the endoprosthesis 1a includes a plurality of inter-connectors 50 to connect adjacent annular elements 10a-10b or 10c-10d. Each inter-connector 50 includes a first bending member or shock 52 and a second bending member or shock 54, which can bend toward each other to separate the adjacent annular elements 10a-10b or 10c-10d or bend away from each other to bring adjacent annular elements closer together. Accordingly, the inter-connector 50 includes a first bending point 60 opposite of a second bending point 64. The first bending member or shock 52 has at least a first arm 66 and a second arm 67. The second bending member or shock 54 has at least a first arm 68 and a second arm 69. The inter-connector 50 couples with a first crossbar 20 of a first annular element 10c at a first coupling 62a, and couples with a second crossbar of a second annular element 10d at a second coupling 62b.

The endoprosthesis 1a can be easily deployed because of the improved flexibility provided within each annular element 10 or between adjacent annular elements 10a-10b. As such, the resiliently-flexible bending members or shocks 52, 54 cooperate to enable the endoprosthesis 1a to bend around a tight corner by the bending members or shocks on one side of the annular element contracting while bending members or shocks on an opposite side expanding. Also, the combination of elbows 30, foot extensions 40, and/or resiliently flexible inter-connectors 50 allow for radial, longitudinal, torsional, or bending loading to be absorbed without cracking, fracturing or damage occurring to the endoprosthesis 1a. Moreover, the resiliently-flexible inter-connectors 50 allow adjacent annular elements to move independently with respect to each other in radial, longitudinal, and cross directions.

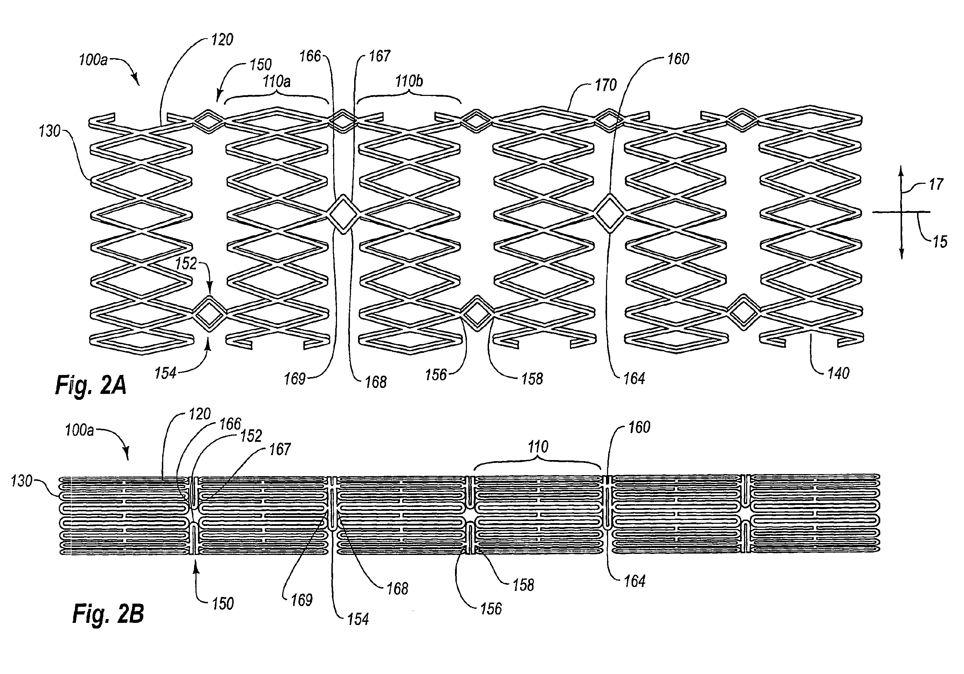

Figures 2A-2B provide side views of an embodiment of another endoprosthesis 100a in a deployed orientation (e.g., Figure 2A) and a delivery orientation (e.g., Figure 2B. The discussions related to endoprosthesis 1a .- also apply to endoprosthesis 100. Accordingly, the endoprosthesis 100a includes a plurality of annular elements 110 that have a plurality of crossbars 120 that are connected together by elbows 130 and intersections 140. More particularly, circumferentially-adjacent crossbars 120 are coupled at an elbow 130 and four or more circumferentially-adjacent crossbars 120 are coupled together at an intersection 140. With this configuration, crossbars 120, intersections 140, and elbows 130 cooperate to form a structure 170 that allows for flexibility as the structure expands or collapses. In the illustrated configuration, the structure 170 has a generally diamond shape that provides the identified flexibility to the endoprosthesis 100. Thus, each annular element 110 has a series of circumferentially-interconnected flexible structures 170, such as, but not limited to, diamond structures, that can expand or collapse under the influence of a balloon or change of temperature.

It will be understood that structure 170 can have other configurations while providing flexibility to the endoprosthesis 100. For instance, structure 170 could be replaced with a repeating "V", a repeating "U", or other structures such as those shown in US Patent No. 6,602,285 and US Patent No. 7,128,756.

Figure 2A shows the endoprosthesis in an expanded orientation so that the annular elements 110 extended away from each other. The adjacent annular elements 110a-110b are separated by members 150, having a generally diamond-shaped configuration in the illustrated configuration, which aid in reducing the forces applied between adjacent annular elements 110. In one sense, the members 150 function as shock absorbing members to aid in and enable movement of adjacent annular elements 110 one with another. The particular configuration of member 150 allows for the annular elements to flex with respect to each other in the longitudinal, radial, and circumferential directions. In part, this is accomplished with the diamond shape configuration having at least two flexing points 160, 164 and at least two couplings 156, 158, although other configurations of the members 150 can also achieve the desired functionality. The two couplings 156, 158 couple two bending member or shocks 152, 154 between adjacent annular elements 110a-110b. The first bending member or shock 152 has a first arm 166 coupled to a second arm 167 through the first flexing point 160, and the second bending member or shock 154 has a first arm 168 coupled to a second arm 169 through the second flexing point 164. The first flexing point 160 cooperates with the second flexing point 164 and the couplings 156, 158 to allow the first annular element 110a to flex and/or move with respect to the second annular element 110b. Moreover, the members 150 cooperate with the elements or structures defining the structure 170 of the annular elements 110 so that the endoprosthesis 100 can bend and flex in any direction.

Figure 2B shows the endoprosthesis 100a in a collapsed orientation so that the annular elements 110 are contracted toward each other for deployment. Accordingly, the adjacent annular elements 110a-110b can be pulled together by the member 150. In the contracted position, the member 150 enables the annular elements 110a-110b to flex with respect to each other in the longitudinal, and cross directions; however, the member 150 can inhibit the collapsed endoprosthesis 100a from flexing in the radial or circumferential directions. This allows the collapsed orientation to enable the endoprosthesis 100a to flex and bend without causing the annular elements 110 to expand or open. In part, this is because the two couplings 156, 158 allow the members 150 to flex independently of the annular elements 110a-110b. Thus, each group of members 150 having the diamond shape can open and close independently during delivery so that the annular elements 110a-110b can move independently around tight corners without incurring undue stress. A further advantage of the member 150 is that member 150 collapses longitudinally during crimping and when disposed within a delivery system, thereby providing column stiffness to push the stent out of the delivery system upon deployment.

### B. Decouplable Endoprosthesis

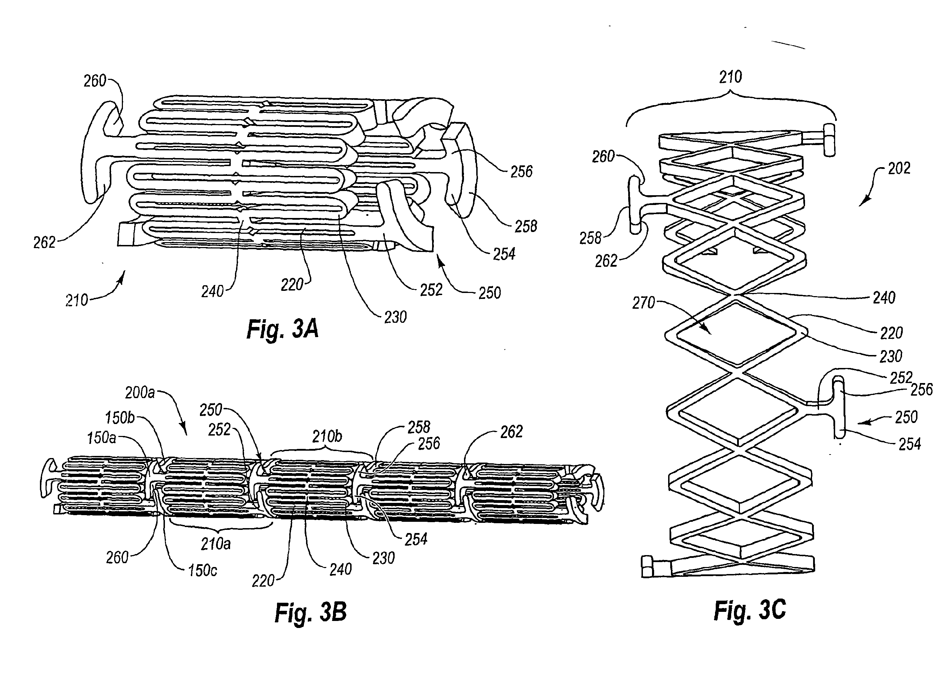

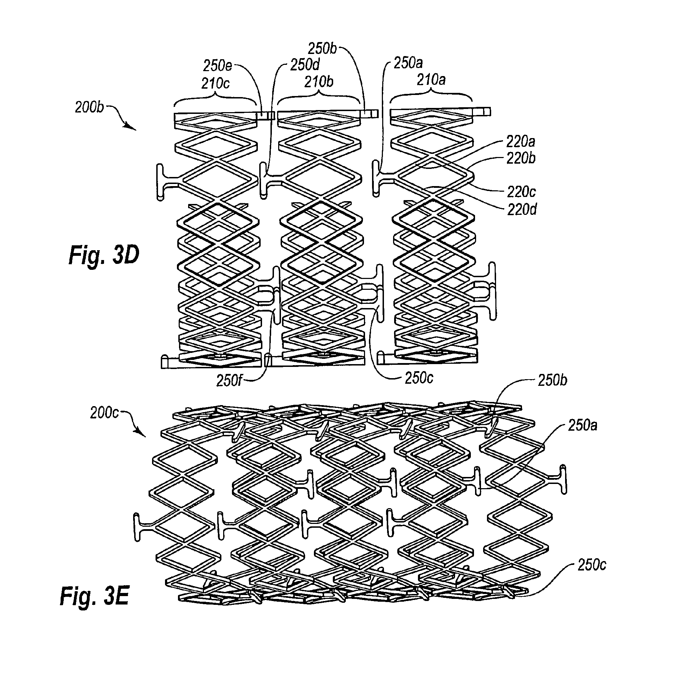

Turning to Figures 3A-3E, illustrated is another configuration of an endoprosthesis that can flex during deployment. In addition, the endoprosthesis, identified by reference numeral 200, can separate into individual sub-endoprostheses after being deployed. These sub-endoprostheses can be considered as individual annular elements that can be interconnected by a plurality of decouplable inter-connectors. The inter-connectors can allow the individual sub-endoprostheses or annular elements to flex, move longitudinally, and/or bend with respect to each other while in a collapsed configuration. Additionally, the inter-connectors can allow the individual sub-endoprostheses to separate away from each other when the primary endoprosthesis is being expanded radially. Thus, the primary endoprosthesis, i.e., the collection of one or more sub-endoprosthesis, can decouple when delivered into a plurality of sub-endoprostheses when at the deployment site within a body lumen.

Figures 3A-3E provide various views of the endoprosthesis 200. For instance, Figures 3A-3B illustrate one configuration of the endoprosthesis 200 in a delivery orientation, while Figures 3C-3E illustrate one configuration of the endoprosthesis in a deployed orientation. As such, all elements described in connection with Figures 3A-3E are intended to be included in each of Figures 3A-3E. The endoprosthesis 200 can include a plurality of annular elements 210 that can each include a plurality of crossbars 220 that are connected together by elbows 230 and intersections 240. Also, the annular elements can be configured as described herein or skilled in the art in light of the teaching contained herein

In the illustrated configuration, circumferentially-adjacent crossbars 220 can be coupled at an elbow 230 and four or more circumferentially- and longitudinally-adjacent crossbars can be coupled together at an intersection 240. The intersection 240 and elbows 230 that connect four crossbars 220a-d can cooperate so as to form a structure 270 that allows for flexibility as the structure can expand or collapse. As illustrated, the structure 270 has a diamond shape, but it will be understood that other configurations are possible so long as they provide the desired flexibility to the endoprosthesis. For instance, structure 270 could be replaced with a repeating "V", repeating "U", wave or WZ structure, or other structures such as those shown in US Patent No. 6,602,285 and US Patent No. 7,128,756. Thus, each annular element 210 can be a sub-endoprosthesis that can have a series of circumferentially-interconnected flexible structure 270 that can flex radially or circumferentially.

Figure 3A shows a configuration of a sub-endoprosthesis 210 in a collapsed orientation so that the crossbars 220 are collapsed toward each other so as to collapse each of the structure 270. The elbows 230 and intersections 240 flex or bend so as to collapse each structure 270. Additionally, the sub-endoprosthesis 210 can include one or more decouplable inter-connectors 250. Each inter-connector 250 can be coupled to an elbow 230 or other portion of the sub-endoprosthesis 210 through a neck 252 that longitudinally extends the inter-connector. The inter-connector 250 can have a first arm 254 and a second arm 256 so as to form a T-shape with the neck 252. The first arm 254 and second arm 256 can combine to form a bumper surface 258. The first arm 254 and second arm 256 can also have a first surface 260 and a second surface 262, respectively, which can engage the corresponding surfaces 260 and 262 of an adjacently positioned one or more inter-connectors 250. This engagement can be achieved through friction fit, mechanical engagement, or other techniques known to those skilled in the art in light of the teaching contained herein.

Figure 3B shows the endoprosthesis 200a in a collapsed orientation so that the sub-endoprostheses 210 are contracted and held together for deployment. In contrast, Figure 3C illustrates the sub-endoprosthesis in an expanded and deployed orientation, which illustrates the inter-connectors 250 circumferentially separating away from each other. With continued reference to Figure 3B, the adjacent annular elements 210a-210b can be held together by the decouplable inter-connectors 250. An inter-connector 250a of a first sub-endoprosthesis 210a can be releasably-coupled with two inter-connector 259b-250c of a second sub-endoprosthesis 210b. As such, the arms 254, 256 of the first sub-endoprosthesis 200b interlock with the arms of the second sub-endoprosthesis 200a. The arms 254, 256 can be releasably-interlocked together by each having friction surfaces 260, 262 that can slide and separate from each other so that the sub-endoprostheses 210a-210b can move relative to each other.

When the endoprosthesis 200a is in the contracted position, the inter-connector 250 enable the sub-endoprostheses 210a-210b to be coupled together and to flex with respect to each other in longitudinal and cross directions. Also, this allows the collapsed orientation to enable the endoprosthesis 200a to flex and bend without causing any of the sub-endoprostheses 210 to expand or open. In part, this is because the inter-connectors 250 allow the sub-endoprosthesis 210 to move independently with respect to each other, thus bending forces generated during tracking or delivery in one of the segments will not be transmitted to adjacent segments. Thus, each inter-connector 250 can move independently during deployment by the surfaces 260 and/or 262 sliding with respect to each other or separating so that the sub-endoprostheses 210a-210b can move independently around tight corners without incurring undue stress.

Figures 3D-3E illustrate portions of the endoprosthesis 200b of Figure 3B in an expanded and deployed orientation. As such, the adjacent sub-endoprostheses 210a-210c can be separated by the decouplable inter-connectors 250 decoupling from each other. More particularly, the inter-connector 250a can decouple from inter-connector 250b and inter-connector 250c, and inter-connector 250d can decouple from inter-connector 250e and inter-connector 250f so as to separate sub-endoprosthesis 210b from the longitudinally-adjacent sub-endoprostheses 210a and 210c. As such, the sub-endoprostheses 210a-210c can move with respect to each other in longitudinal, radial, cross, and circumferential directions. In essence, the endoprosthesis 200b is deployed into a plurality of separate and distinct sub endoprostheses 210a-210c.

### C. Bumper Endoprosthesis

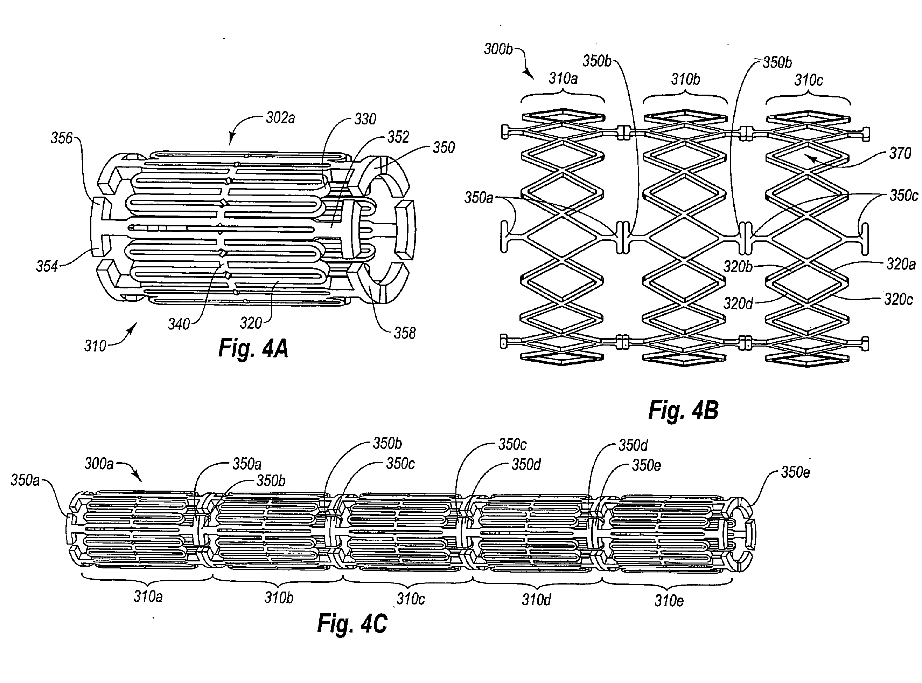

Figures 4A-4C illustrate another configuration of an endoprosthesis that can flex during deployment and separate into individual sub-endoprostheses after being set. These sub-endoprostheses can be positioned adjacent to and in contact with each other when in a collapsed orientation and separate from each other when opened or expanded into a deployed orientation. The sub-endoprostheses can include bumpers that allow longitudinal forces to be transmitted throughout a portion or the entire endoprosthesis, thereby allowing the sub-endoprostheses to flex, move longitudinally, and/or bend with respect to each other while in a collapsed configuration. The bumpers may also be configured to prevent transmission of rotational forces between the adjacent segments as will be described in detail below. Additionally, the bumpers can allow the individual sub-endoprostheses to separate away from each other when the primary endoprosthesis, i.e., the collection of one or more sub-endoprosthesis, is being expanded radially. Thus, the primary endoprosthesis can decouple into a plurality of sub-endoprostheses when at the deployment site within a body lumen.

Figures 4A-4C provide various views of a sub-endoprosthesis 300, including the endoprosthesis 300a in a delivery orientation. As such, all elements described in connection with Figures 4A-4C are intended to be included in each of Figures 4A-4C. The endoprosthesis 300 can include a plurality of annular elements 310 that can each have a plurality of crossbars 320 that are connected together by elbows 330 and intersections 340. Also, the annular elements 310 can be configured as described herein or as is well known in the art. In the illustrated configuration, circumferentially-adjacent crossbars 320 can be coupled at an elbow 330 and two or more circumferentially- and longitudinally-adjacent crossbars can be coupled together at an intersection 340. The intersection 340 and elbows 330 that connect four crossbars 320a-d can cooperate so as to form a structure 370 that can expand or collapse while providing sufficient scaffolding to the vessel wall. Thus, each annular element 310 is a sub-endoprosthesis that can have a series of circumferentially-interconnected flexible structures 370 that can flex radially or circumferentially. Although the illustrated structure 370 has a diamond configuration, one skilled in the art will appreciate that the structure 370 can have various other configurations so long as it is capable of providing the desired flexibility. For instance, and not by way of limitation, the structure 370 can have configurations similar to the repeating "V", repeating "U", wave or WZ structure, or other structures such as those shown in US Patent No. 6,602,285 and US Patient, No. 7,128,756.

Figure 4A shows a sub-endoprosthesis 310 in a collapsed orientation so that the crossbars 320 are collapsed toward each other so as to collapse each of the structures 370. More particularly, the elbows 330 and intersections 340 flex or bend so as to collapse each structure 370. Additionally, the sub-endoprosthesis 310 can include one or more bumpers 350. Each bumper 350 can be coupled to an elbow 330 or other portion of the sub-endoprosthesis 310 through a neck 352 that longitudinally extends the bumper. The bumper 350 can have a first arm 354 and a second arm 356 so as to form a T-shape with the neck 352. Also, the first arm 354 and second arm 356 can combine to form a bumper surface 358. (Comment: It should be noted that it is also possible to include a bumper that is not connect through a neck. For example, the bumper profile can overlap the stent elbow profile. Also, the elbow itself could be cropped to include a flattened section that operates as a bumper.)

Figure 4C shows the endoprosthesis 300a in an collapsed orientation so that the sub-endoprostheses 310 are contracted and held together for deployment. Accordingly, the adjacent annular elements 310a-310e can be in contact through the bumpers 350a-350e. The bumpers 350a-e allow the sub-endoprostheses 310a-310e to slide and separate from each other so that the sub-endoprostheses can move relative to each other during and after deployment. For instance, the bumpers 350a-e can provide desirably push transmission and axial stiffness while permitting relative motion and independence. Further, the configuration of the bumpers 350a-e can provide some limitation to the movement of adjacent annular elements, while permitting independent movement of the annular element during and after deployment.

When the endoprosthesis 310a is in the contracted position, the bumpers 350 enable the sub-endoprostheses 310a-310b to be held together by a delivery catheter (not shown) that substantially surrounds the endoprosthesis 300a and to move with respect to each other in longitudinal and cross directions. Also, this allows the collapsed orientation to enable the endoprosthesis 300a to flex and bend without causing any of the sub-endoprostheses 310 to expand or open. In part, this is because the bumpers 350 allow the sub-endoprostheses 310 to move independently with respect to each other. Thus, each bumper 350 can move independently during deployment by the bumper surfaces 358 sliding with respect to each other or separating so that the sub-endoprostheses 310a-310e can move independently around tight corners without incurring undue stress.

Figure 4B illustrates a portion of the endoprosthesis 300b of Figure 4C in an expanded and deployed orientation. As such, the adjacent sub-endoprostheses 310a-310c can be separated by the bumpers 350. More particularly, the bumpers 350a of the first sub-endoprosthesis 310a can abut or separate from the bumpers 350b of the second endoprosthesis 310b, and the bumpers 350b can abut or separate from the bumpers 350c of the third endoprosthesis 310C. As such, the deployed sub-endoprostheses 310a-310c can move with respect to each other in the longitudinal, radial, cross, and circumferential directions. In essence, the endoprosthesis 300b is deployed into a plurality of separate and distinct sub-endoprostheses 310a-310c.

### II. Inter-Connectors

The use of interconnections allows for improved overall structural integrity of an endoprosthesis. The interconnections can allow for adjacent annular elements of an endoprosthesis to be delivered together and to separate into distinct sub-endoprostheses during expansion into a deployed orientation. As such, the interconnections can inhibit the endoprosthesis from cracking at high stress areas through reducing the stress upon the high stress areas, thereby improving the performance and reliability of the endoprosthesis. The interconnections can be configured as decouplable inter-connectors and/or bumpers, as described above.

### A. Decouplable Inter-Connectors

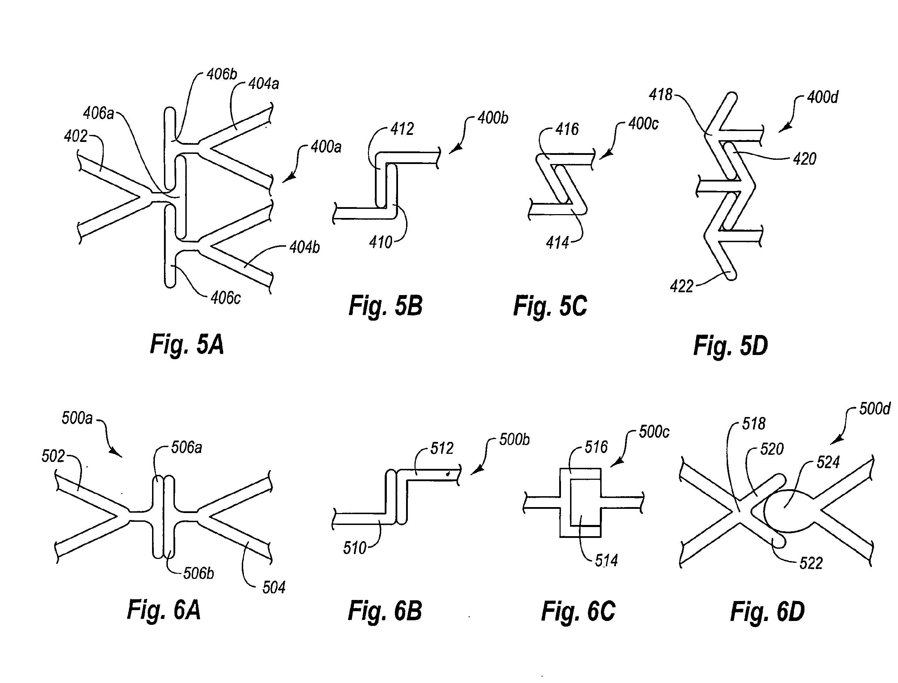

The decouplable inter-connectors described in Figures 3A-3E can be prepared in a variety of configurations as shown in Figures 5A-5D as well as others known to those skilled in the art in light of the teaching contained herein. As such, Figure 5A depicts a configuration of an interconnection system 400a that releasably-couples a pair of longitudinally-adjacent sub-endoprostheses 402, 404. Accordingly, sub-endoprosthesis 402 includes a T-shaped decouplable inter-connector 406a that cooperates and releasably-couples with the T-shaped decouplable inter-connectors 406b, 406c of sub-endoprostheses 404a, 404b, respectively. Figure 5B depicts a configuration of a interconnection system 400b that uses L-shaped decouplable inter-connectors 410, 412. Figure 5C depicts a configuration of an interconnection system 400c that uses V-shaped decouplable inter-connectors 414, 416. Figure 5D depicts a configuration of an interconnection system 400d that uses anchor-shaped decouplable inter-connectors 418, 420, 422.

### B. Bumpers

The bumpers described in Figures 4A-4C can be prepared in a variety of configurations as shown in Figures 6A-6D as well as others. As such, Figure 6A depicts a configuration of a bumper system 500a that allows a pair of longitudinally-adjacent sub-endoprostheses 502, 504 to come into contact during deployment without imparting unfavorable stress or strains. Accordingly, sub-endoprosthesis 502 can include a T-shaped bumper 506a that can abut or separate from the T-shaped bumper 506b of sub-endoprosthesis 404. Figure 6B depicts a configuration of a bumper system 500b that uses L-shaped bumpers 510, 512. Figure 6C depicts a configuration of a bumper system 500c that uses an extended element 514 and a receiver element 516; the extended element 514 selectively; releasably fitting into the receiver element 516. Figure 6D depicts a configuration of a bumper system 500d that uses a rounded extended element 524 and a guide element 518; the rounded extended element 524 can slide against either guide arm 520, 522 so as to be received into the guide element 518. Through the use of bumper systems 500c and 500d relative rotational motion of adjacent annular elements is controlled due to frictional contact between the extended or guide element with the receiver or rounded element.

### III. Compression Endoprosthesis

Additionally, an endoprosthesis can be configured to have column stiffness during delivery and flexibility after being expanded or deployed. The stiffness can be achieved with compressible inter-connectors that allow adjacent annular elements to collapse against each other. Optionally, an overlapping region can allow circumferentially-adjacent compressible inter-connectors to overlap while in the delivery orientation so as to create a physical barrier between adjacent annular elements. Also, the overlapping region can release when the endoprosthesis expands so as to create space for the adjacent annular elements to flex or move longitudinally with respect to each other.

A compressible endoprosthesis can have inter-connectors that compress or lengthen when bent, stretched, or shortened. The compressible inter-connectors can absorb longitudinal or bending stresses to allow the rings to maintain uniformity, which improves their resistance to cracking or failure that may occur due to various loading conditions arising from stresses that occur during longitudinal, bending, and radial cyclical loading. Optionally, the compressible inter-connectors can have a member that overlaps another member of a circumferentially adjacent compressible inter-connector so as to create a rigid section between adjacent annular elements. The compressible inter-connectors featuring the overlapping portions can be included in the described endoprostheses as well as other configurations well known in the art. For example, compressible inter-connector can include a compressible arm having a V-shape that has one side connected to one annular element, and a second side that is connected to a second annular element through an extension arm. Additionally, each of the arms of the V-shape and extension arm can be long to increase flexibility. Moreover, the point of the V-shape can be cradled within the opening of an adjacent inter-connector so as to form the overlapping configuration.

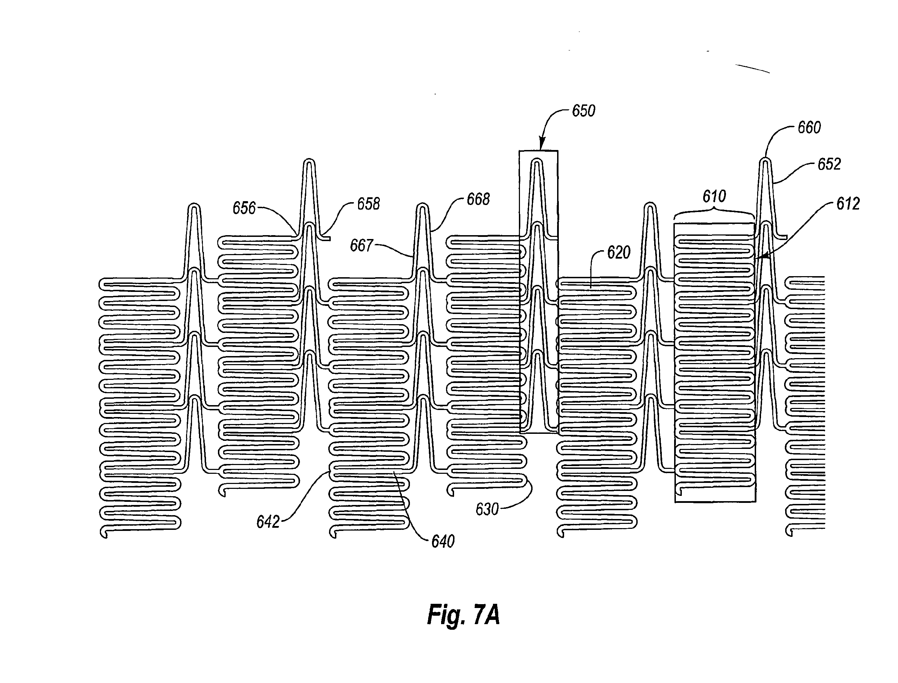

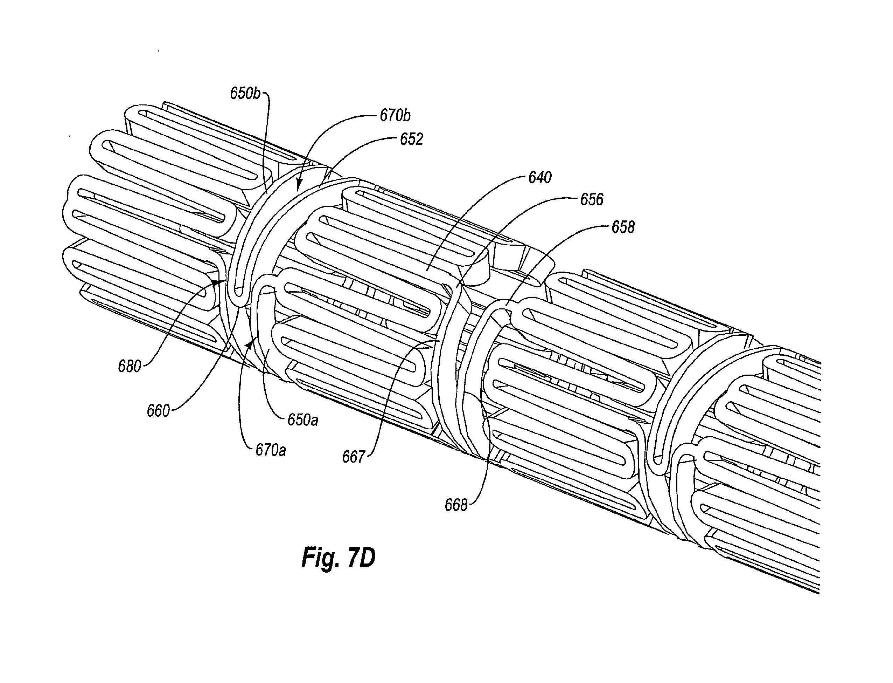

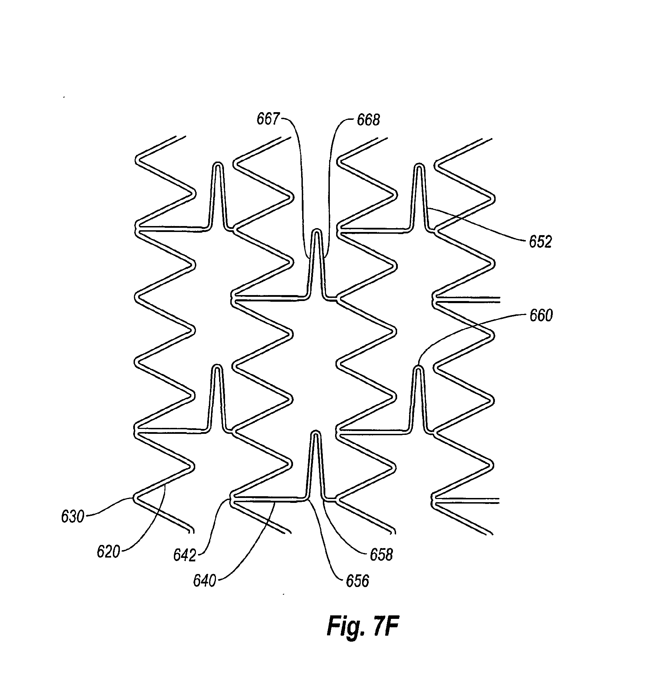

One configuration can include an endoprosthesis configured to be compressed during deployment and be compressible after being expanded and set. Figures 7A-10B illustrate endoptiostheaes that include a plurality of annular elements that are interconnected by compression inter-connectors that can function as shock absorbers. The compression inter-connectors configured as shock absorbers, or means for reducing force transmission between adjacent annular elements, allow the individual annular elements to flex, move longitudinally, and/or bend with respect to each other while in a collapsed or deployed configuration. Additionally, the compression inter-connectors can allow the individual annular elements to flex radially, circumferentially, axially, and longitudinally while deployed.

### A. Compressible V-Shaped Inter-Connector

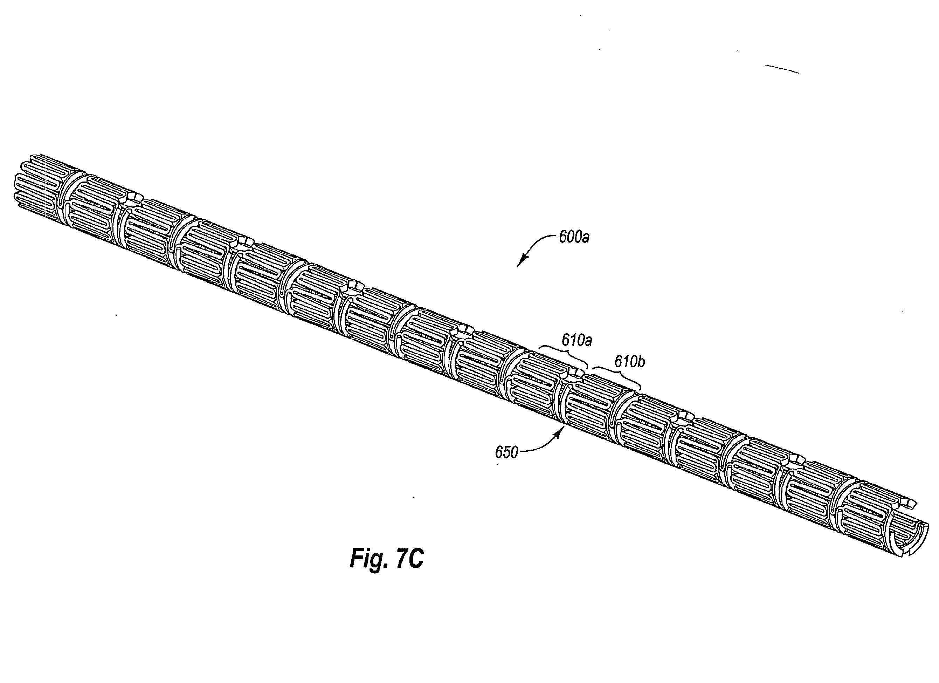

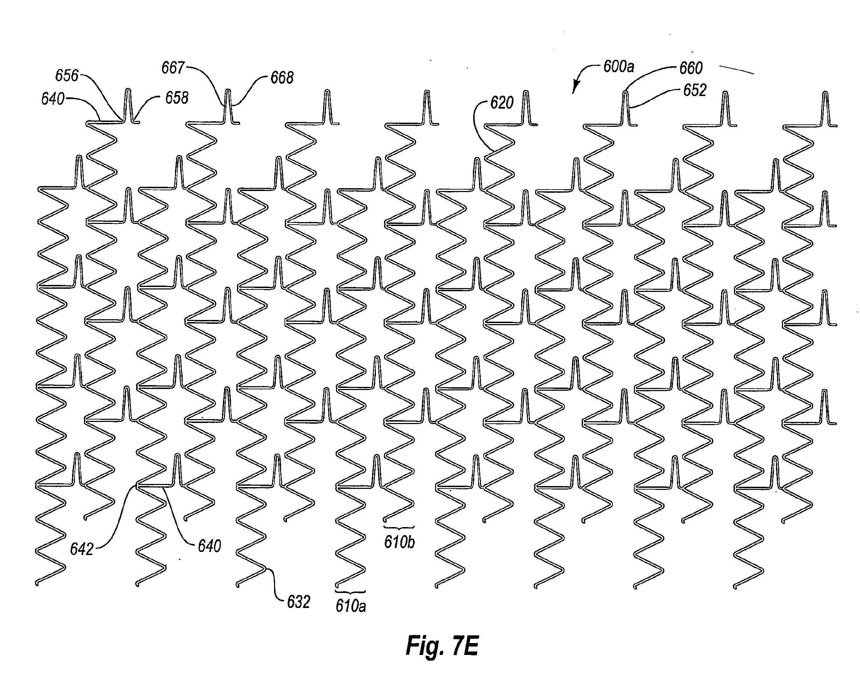

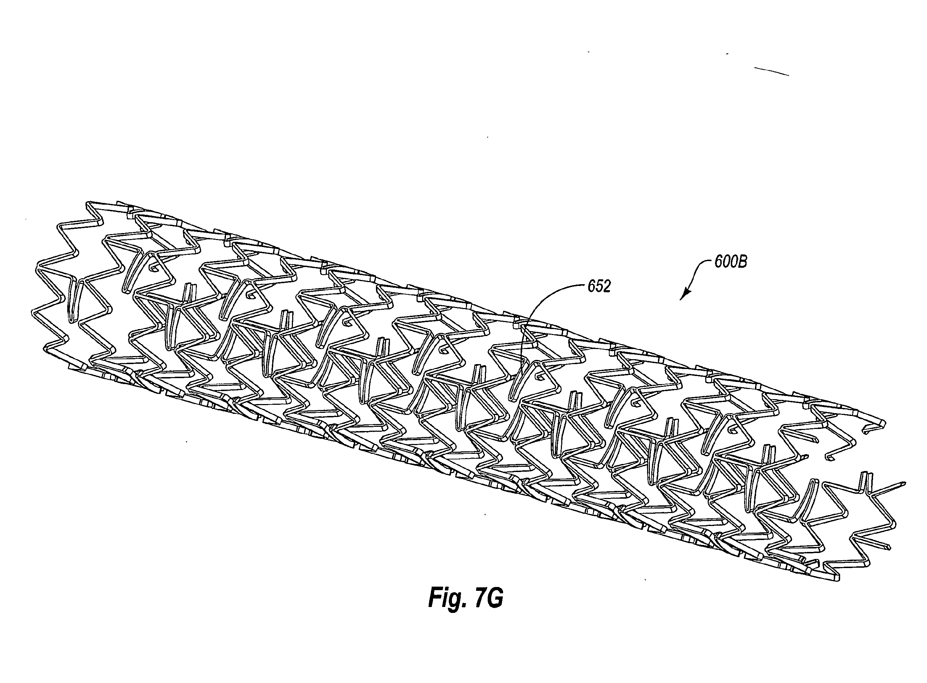

Figures 7A-7H provide different views of a collapsible endoprosthesis 600 in a delivery orientation (e.g., flat planar view shown in Figures 7A-7B; tubular shown in Figures 7C-7D) and a deployed orientation (e.g., flat planar shown in Figures 7E-7F; tubular shown in Figures 7G-7H). The discussions related to endoprosthesis 1a can also apply to endoprosthesis 600. Accordingly, the endoprosthesis 600 can include a plurality of annular elements 610 that can have a plurality of crossbars 620 that are connected together by elbows 630. More particularly, circumferentially-adjacent crossbars 620 can be coupled at an elbow 630 that can be configured as a repeating serpentine pattern. With this configuration, crossbars 620 and elbows 630 can cooperate so as to form a structure 612, such as a repeating serpentine pattern or other structure, that allows for flexibility as the structure can expand or collapse. In the illustrated configuration, the structure 612 has a generally serpentine shape that can provide the identified flexibility to the endoprosthesis 600; however, the structure 612 can be an other configurations, such as a diamond-shape, V-shape, U-shape, W-shape, O-shape, N-shape, M-shape, Z-shape, and the like. Thus, each annular element 610 can have a series of circumferentially-interconnected flexible structures 612 that can expand or collapse under the influence of a balloon or change of temperature.

It will be understood that structure 612 can have other configurations while providing flexibility to the endoprosthesis 600. For instance, structure 612 could be replaced with an, repeating "V", repeating "U", wave or WZ structure as described herein. Additionally, Figures 7A-7H show different view of an endoprosthesis 600 that includes a plurality of the compression inter-connectors 650 that couple adjacent annular elements 610a-610b. The compression inter-connectors 650 are generally configured to include an extension arm 640 that extends a collapsing arm 652 from an elbow 630. As such, the extension arm 640 is coupled to the elbow 630 through an extension coupling 642, which can be flexible as in the other couplings described herein. The flexible extension coupling 642 and extension arm 640 can allow for the collapsing arm 652 to freely collapse within a space between adjacent annular elements 610a-610b.

Figure 7A-7B are flat planar views that show the endoprosthesis 600a in a collapsed orientation so that the annular elements 610 are contracted toward each other for delivery. Accordingly, the adjacent annular elements 610a-610b can be pulled together by the compression inter-connectors 650. In the contracted position, the compression inter-connectors 650 enable the annular elements 610a-610b to flex with respect to each other in the longitudinal, and cross directions; however, the compression inter-connectors 650 can inhibit the collapsed endoprosthesis 600a from flexing in the radial or circumferential directions. This allows the collapsed orientation to enable the endoprosthesis 600a to flex and bend without causing the annular elements 610 to expand or open. In part, this is because the two couplings 656, 658 allow the compression inter-connectors 650 to flex independently of the annular elements 610a-610b. Thus, each group of compression inter-connectors 650 can open and close independently during deployment so that the annular elements 610a-610b can move independently around tight corners without incurring undue stress.

Generally, the compression inter-connectors 650 can have a V-shape configuration having at least one flexing point 660 and at least two flexible couplings 656, 658, although other configurations of the compression inter-connectors 650 can also achieve the desired functionality. The two couplings 656, 658 can couple a collapsing member or shock 652 between adjacent annular elements 610a-610b. The collapsing member or shock 652 can have a first arm 667 coupled to a second arm 668 through the flexing point 660. The flexing point 660 can cooperate with the two couplings 656, 658 to allow the first annular element 610a to flex and/or move with respect to the second annular element 610b. Moreover, the compression inter-connectors 650 can cooperate with the annular elements 610 so that the endoprosthesis 100 can bend and flex in any direction. For example, the compression inter-connectors 650 can be shaped as diamond-shape, V-shape, U-shape, W-shape, O-shape, N-shape, M-Shape, Z-shape, and the like.

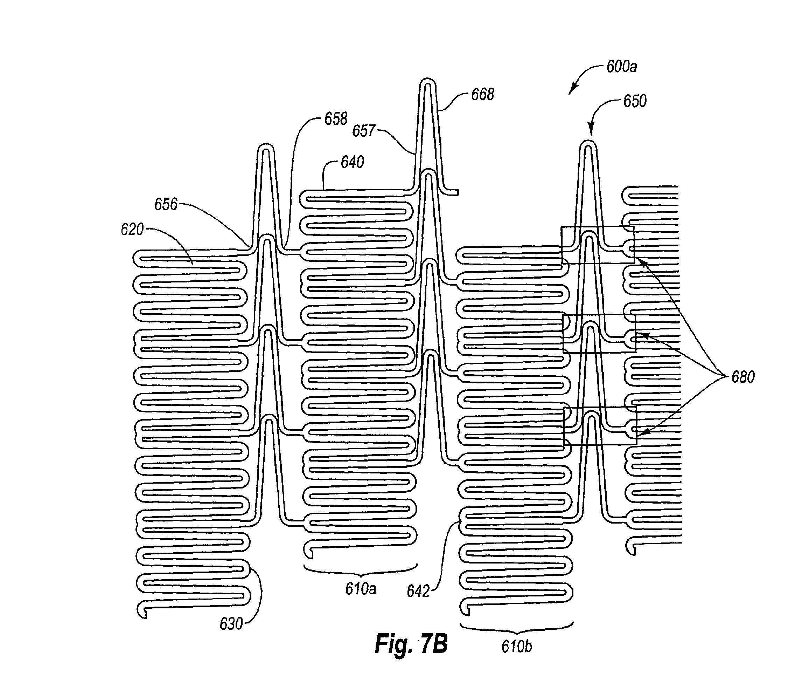

Additionally, Figure 7B shows the collapsed configuration of the endoprosthesis 600a to have an overlapping region 680 that allows circumferentially-adjacent compression inter-connectors 650 to overlap. As such, the overlapping region 680, which is essentially a flexing point 660 overlapping the two coupling 656, 658, provides the segmented endoprosthesis 600a with additional strength when in the delivery configuration, especially when in a catheter.

Figures 7C-7D are perspective tubular views of the endoprosthesis 600a of Figures 7A-7B. These views show that a first arm 667 and second arm 668 of a first compression inter-connector 650a can cooperate to form a space 670a. As such, the flexing point 660 of the second compression inter-connector 650b, which is circumferentially-adjacent to the first compression inter-connector 650a, can fit within the space 670a. Moreover, the second compression inter-connector 650 can likewise form a second space 670b for the next sequentially-adjacent compression inter-connector 650.

Figures 7E-7F are planar side views that show the endoprosthesis 600a in an expanded or deployed orientation so that the annular elements 610 are extended away from each other. The adjacent annular elements 610a-610b can be separated by compression inter-connectors 650, having the V-shaped configuration with the extension arm 642 (or others) in the illustrated configuration, which aid in reducing the forces applied between adjacent expanded annular elements 610. In one sense, the compression inter-connectors 650 function as shock absorbing members to aid in and enable movement of adjacent expanded annular elements 610 one with another. The particular configuration of compression inter-connectors 650 can allow for the annular elements to flex with respect to each other in the longitudinal, radial, and circumferential directions. In part, this can be accomplished with the V-shape configuration having at least one flexing point 660 and at least two flexible couplings 656, 658, although other configurations of the compression inter-connectors 650 can also achieve the desired functionality. Thus, the two couplings 656, 658 that can couple a collapsing member or shock 652 between adjacent annular elements 610a-610b can allow expanded annular elements to move freely with respect to each other.

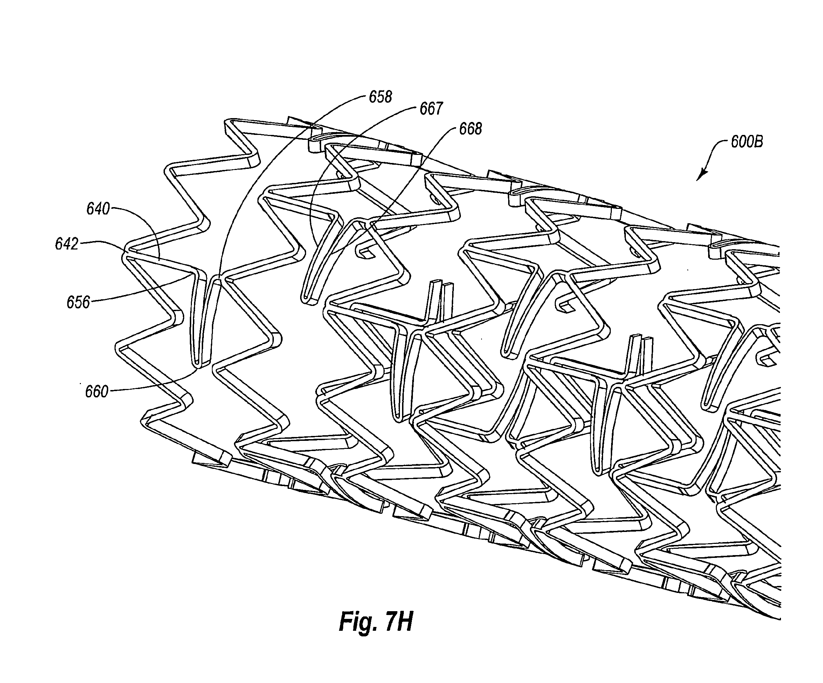

Figures 7G-7H are perspective tubular views of the expanded endoprosthesis 600b of Figures 7E-7F. More particularly, Figure 7G shows the endoprosthesis 600b being expanded so as to achieve the diameter of a vessel or other lumen. Figure 7H shows a magnified view of Figure 7G, which allows the elements of the endoprosthesis 600b to be easily viewed while in a deployed orientation.

### B. Compressible Z-Shaped Inter-Connector

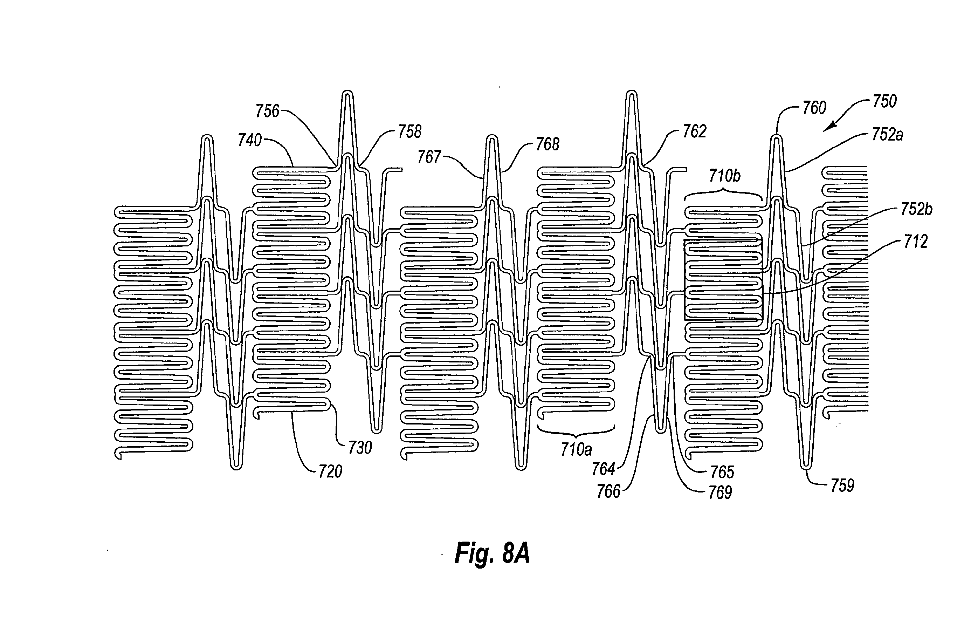

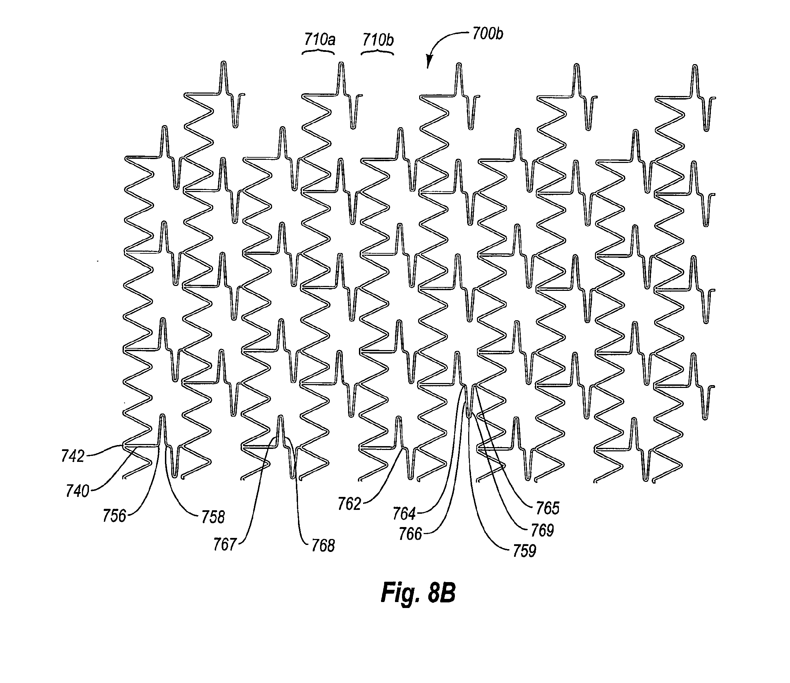

Figures 8A-8C provide different views of a collapsible endoprosthesis 700 in a delivery orientation (e.g., Figure 8A), a deployed orientation (e.g., Figures 8B-8C). The discussions related to endoprosthesis 1a can also apply to endoprosthesis 700. Accordingly, the endoprosthesis 700 can include a plurality of annular elements 710 that can have a plurality of crossbars 720 that are connected together by elbows 730. More particularly, circumferentially-adjacent crossbars 720 can be coupled at an elbow 730 that can be configured as a repeating serpentine pattern. With this configuration, crossbars 720 and elbows 730 can cooperate so as to form a structure 712, such as a repeating serpentine pattern or other structure, that allows for flexibility as the structure can expand or collapse. In the illustrated configuration, the structure 712 has a generally serpentine shape that can provides the identified flexibility to the endoprosthesis 700; however, the structure 712 can be an other configurations, such as a diamond-shape, V-shape, U-shape, W-shape, O-shape, N-shape, M-shape, Z-shape, and the like. Thus, each annular element 710 can have a series of circumferentially-interconnected flexible structures 712 that can expand or collapse under the influence of a balloon or change of temperature.

It will be understood that structure 712 can have other configurations while providing flexibility to the endoprosthesis 700. For instance, structure 712 can have a configuration similar to those described herein and in US Patent No. 6,602,285 and US Patent No. 7,128,756.

Additionally, Figure 8A shows an endoprosthesis 700 that includes a plurality of the compression inter-connectors 750 that couple adjacent annular elements 710a-710b. The compression inter-connectors 750 are generally configured to include an extension arm 740 that extends a pair of coupled collapsing arms 752a-752b from an elbow 730. As such, the extension arm 740 is coupled to the elbow 730 through an extension coupling 742, which can be flexible as in the other couplings described herein. The flexible extension coupling 742 and extension arm 740 can allow for the collapsing arms 752a-752b to freely collapse within a space between adjacent annular elements 710a-710b.

Figure 8A is a flat planar view that shows the endoprosthesis 700a in a collapsed orientation so that the annular elements 710 are contracted toward each other for deployment. Accordingly, the adjacent annular elements 710a-710b can be pulled together by the compression inter-connectors 750. In the contracted position, the compression inter-connectors 750 enable the annular elements 710a-710b to flex with respect to each other in the longitudinal, and cross directions; however, the compression inter-connectors 750 can inhibit the collapsed endoprosthesis 700a from flexing in the radial or circumferential directions. This allows the collapsed orientation to enable the endoprosthesis 700a to flex and bend without causing the annular elements 710 to expand or open. In part, this is because the two couplings 756, 758 of a first collapsing arm 752a and the two couplings 764, 765 of a second collapsing arm 752b allow the compression inter-connectors 750 to flex independently of the annular elements 710a-710b. Thus, each group of compression inter-connectors 750 can open and close independently during deployment so that the annular elements 710a-710b can move independently around tight corners without incurring undue stress.

Generally, the compression inter-connectors 750 can have substantially a Z-shape configuration having at least one flexing point 760 and at least two flexible couplings 756, 758 for a first collapsing arm 752a, and at least one flexing point 759 and at least two flexible couplings 764, 765 for a second collapsing arm 752b. Preferably, the first collapsing arm 752a is oriented oppositely from the second collapsing arm 752b so that the flexing points 760, 759 point away from each other. However, other configurations of the compression inter-connectors 750 can also achieve the desired functionality. The first collapsing arm 752a can be comprised of a first arm 767 and a second arm 768 that are coupled together through a flexing point 760. The first arm 767 can be coupled to the extension arm 740 through a first coupling 756, and the second arm 768 can be coupled to the second collapsing arm 752b through a second coupling 758. The second collapsing arm 752b can be comprised of a first arm 766 and a second arm 769 that are coupled together through a flexing point 759. The first arm 766 of the second collapsing arm 752b can be coupled to the first collapsing arm 752a through a first coupling 764, and the second arm 769 of the second collapsing arm 752b can be coupled to the elbow 730 of an adjacent annular element 710. More particularly, the second coupling 758 of the first collapsing arm 752a and the first coupling 764 of the second collapsing arm 752b can be coupled together through a flexible transition coupling 762.

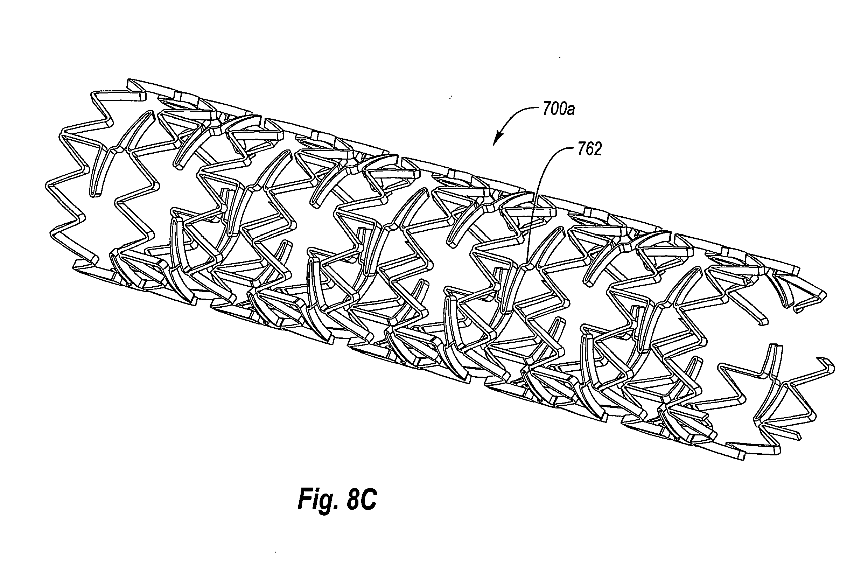

Additionally, Figure 8B is a planar side view that shows the endoprosthesis 700b in an expanded or deployed orientation so that the annular elements 710 are extended away from each other, while Figure 8C is a perspective view of the tubular endoprosthesis in the expanded or deployed orientation. With continued reference to Figure 8B, in one sense, the compression inter-connectors 750 function as shock absorbing members to aid in and enable movement of adjacent expanded annular elements 710 one with another. The particular configuration of compression inter-connectors 750 can allow for the annular elements to flex with respect to each other in the longitudinal, radial, and circumferential directions. In part, this can be accomplished with the serpentine shape configuration having at least a first collapsing arm 752a that includes a flexing point 760 and at least two flexible couplings 756, 758, and a second collapsing arm 752b that includes a flexing point 759 and at least two flexible couplings 764, 765. However, other configurations of the compression inter-connectors 650 can also achieve the desired functionality. Thus, the Z-shaped compression inter-connectors 750 between adjacent annular elements 610a-610b can allow expanded annular elements to move freely with respect to each other.

### C. Offset Inter-Connectors

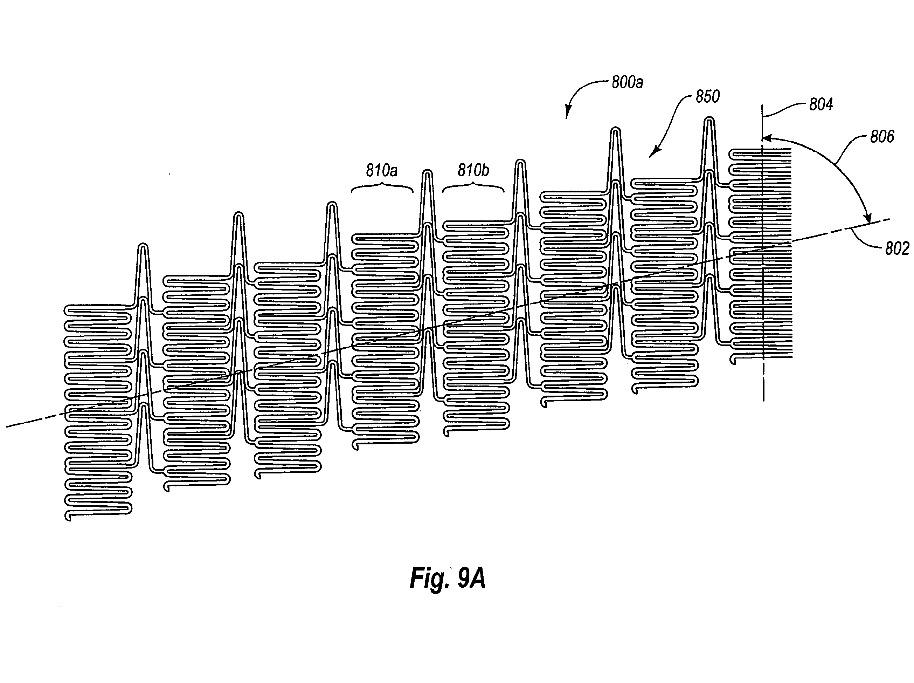

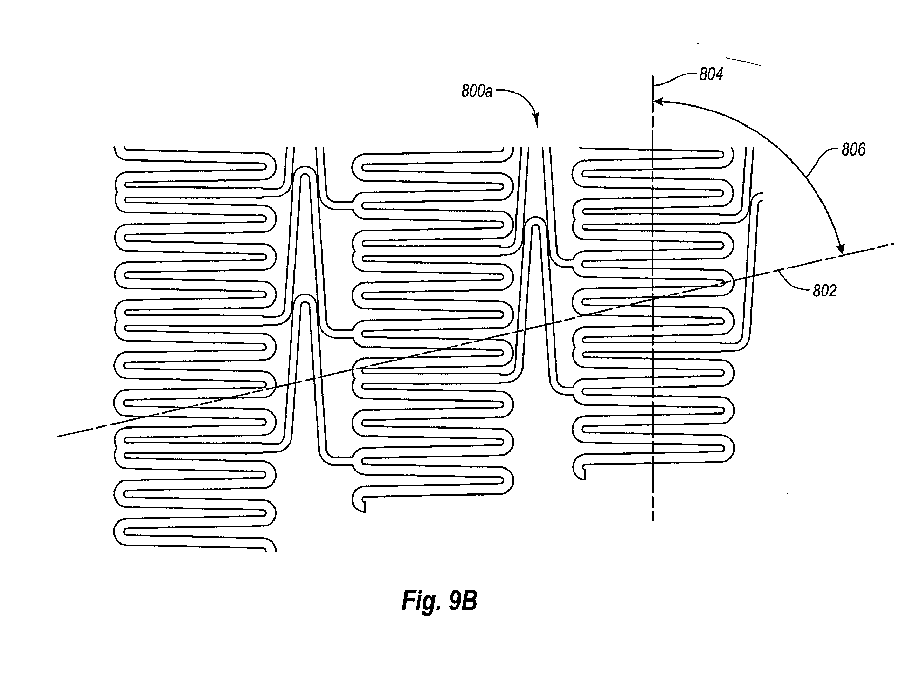

Figures 9A-9B are planar side views of a coiled endoprosthesis 800a having an offset configuration. More particularly, Figure 9A shows the endoprosthesis 800a in a collapsed or delivery configuration, and Figure 9B shows a magnified view of Figure 9A. The endoprosthesis can include longitudinally-adjacent annular elements 810a-810b that are interconnected by compression inter-connectors 850. Also, the longitudinally-adjacent annular elements 810a-810b can be a continuous helical element that wraps around a central, longitudinal axis 802. Accordingly, the first annular element 810a continues to second annular element 810b by being in an offset direction 804. This can be seen with the annular elements 810 having an offset direction 804 that intersects the longitudinal axis 802 at an angle 806. Preferably, the angle 806 is not orthogonal to the longitudinal axis 802. Thus, the adjacent annular elements 810a-810b are a continuous coil that are additionally coupled through compression inter-connectors 750. As such, any compression-inter-connectors 750 described herein can be used with a coiled endoprosthesis 800a, and the coiled endoprosthesis can function as the endoprostheses 600-700 described above.

### IV. Endoprosthetic Composition

The endoprostheses of the present invention can be made of a variety of materials, such as, but not limited to, those materials which are well known in the art of endoprosthesis manufacturing. This can include, but not limited to, an endoprosthesis having a primary material for both the annular elements and inter-connectors. Alternatively, the inter-connectors can be made of a different material to the annular element. Generally, the materials for the endoprosthesis can be selected according to the structural performance and biological characteristics that are desired.

In one configuration, the inter-connectors and/or the annular elements have multiple layers, with at least one layer being applied to a primary material forming the annular elements. The multiple layers on the inter-connectors and/or the annular elements can be resiliently flexible materials or rigid and inflexible materials. For example, materials such as Ti3A12.5V, Ti6A14V, 3-2.5T1, 6-4Ti and platinum may be particularly good choices for adhering to a flexible material, such as, but not limited to, Nitinol and providing good crack arresting properties. The use of resiliently flexible materials can provide shock-absorbing characteristics to the structures, decouplable inter-connectors, and/or bumpers, which can also be beneficial for absorbing stress and strains, which may inhibit crack formation at high stress zones. Also, the multiple layers can be useful for applying radiopaque materials to the endoprosthesis. For example, types of materials that are used to make an endoprosthesis can be selected so that the endoprosthesis is capable of being collapsed during placement and expanded when deployed. Usually, the endoprosthesis can be self-expanding, balloon-expandable, or can use some other well-known configuration for deployment. For purposes of illustration and not limitation, reference is made generally to self-expanding embodiments and balloon-expandable embodiments of the endoprosthesis of the present invention; however, other types of endoprostheses can be configured in accordance with the present invention.

Self-expanding embodiments of an endoprosthesis can include a material made from any of a variety of known suitable materials, such as a shaped memory material ("SMM"). For example, the SMM can be shaped in a manner that allows for restriction to induce a substantially tubular, linear orientation while within a delivery shaft, but can automatically retain the memory shape of the endoprosthesis once extended from the delivery shaft. SMMs have a shape memory effect in which they can be made to remember a particular shape. Once a shape has been remembered, the SMM may be bent out of shape or deformed and then returned to its original shape by unloading from strain or heating. Typically, SMMs can be shape memory alloys ("SMA") comprised of metal alloys, or shape memory plastics ("SMP") comprised of polymers.

Usually, an SMA can have any non-characteristic initial shape that can then be configured into a memory shape by heating the SMA and conforming the SMA into the desired memory shape. After the SMA is cooled, the desired memory shape can be retained. This allows for the SMA to be bent, straightened, compacted, and placed into various contortions by the application of requisite forces; however, after the forces are released, the SMA can be capable of returning to the memory shape. The main types of SMAs are as follows: copper-zinc-aluminium; copper-aluminium-nickel; nickel-titanium ("NiTi") alloys known as nitinol; and cobalt-chromium-nickel alloys or cobalt-chromium-nickel-molybdenum alloys known as elgiloy alloys. The temperatures at which the SMA changes its crystallographic structure are characteristic of the alloy, and can be tuned by varying the elemental ratios.

For example, the primary material of an endoprosthesis can be of a NiTi alloy that forms superelastic nitinol. In the present case, nitinol materials can be trained to remember a certain shape, straightened in a shaft, catheter, or other tube, and then released from the catheter or tube to return to its trained shape. Also, additional materials can be added to the nitinol depending on the desired characteristic.

An SMP is a shape-shifting plastic that can be fashioned into an endoprosthesis in accordance with the present invention. Also, it can be beneficial to include at least one layer of an SMA and at least one layer of an SMP to form a multilayered body; however, any appropriate combination of materials can be used to form a multilayered endoprosthesis. When an SMP encounters a temperature above the lowest melting point of the individual polymers, the blend makes a transition to a rubbery state. The elastic modulus can change more than two orders of magnitude across the transition temperature ("Ttr"). As such, an SMP can formed into a desired shape of an endoprosthesis by heating it above the Ttr, fixing the SMP into the new shape, and cooling the material below Ttr. The SMP can then be arranged into a temporary shape by force, and then resume the memory shape once the force has been applied. Examples of SMPs include, but are not limited to, biodegradable polymers, such as oligo(ε-caprolactone)diol, oligo(p-dioxanone)diol, and non-biodegradable polymers such as, polynorborene, polyisoprene, styrene butadiene, polyurethane-based materials, vinyl acetate-polyester-based compounds, and others yet to be determined. As such, any SMP can be used in accordance with the present invention.

For example, Veriflex™, the trademark for CRG's family of shape memory polymer resin systems, currently functions on thermal activation which can be customizable from -28.9°C to 271.1°C (-20°F to 520°F), allowing for customization within the normal body temperature. This allows an endoprosthesis having at least one layer comprised of Veriflex™ to be inserted into a delivery catheter. Once unrestrained by the delivery shaft, the body temperature can cause the endoprosthesis to return to its functional shape.

An endoprosthesis having at least one layer made of an SMM or suitable superelastic material and other suitable layers can be compressed or restrained in its delivery configuration within a delivery device using a sheath or similar restraint, and then deployed to its desired configuration at a deployment site by removal of the restraint as is known in the art. An endoprosthesis made of a thermally-sensitive material can be deployed by exposure of the endoprosthesis to a sufficient temperature to facilitate expansion as is known in the art.

Balloon-expandable endoprosthesis embodiments can be comprised of a variety of known suitable deformable materials, including stainless steel, silver, platinum, tantalum, palladium, cobalt-chromium alloys or other known biocompatible materials.

For delivery, the balloon-expandable endoprosthesis having suitable materials can be mounted in the delivery configuration on a balloon or similar expandable member of a delivery device. Once properly positioned within the body lumen at a desired location, the expandable member can be expanded to expand the endoprosthesis to its deployed configuration as is known in the art.

Also, balloon endoprosthesis embodiments can include a suitable biocompatible polymer in addition to or in place of a suitable metal. The polymeric endoprosthesis can include biodegradable or bioabsorbable materials, which can be either plastically deformable or capable of being set in the deployed configuration. If plastically deformable, the material can be selected to allow the endoprosthesis to be expanded in a similar manner using an expandable member so as to have sufficient radial strength and scaffolding and also to minimize recoil once expanded. If the polymer is to be set in the deployed configuration, the expandable member can be provided with a heat source or infusion ports to provide the required catalyst to set or cure the polymer. Alternative known delivery devices and techniques for self-expanding endoprostheses likewise can be used.

Additionally, a self-expanding configuration of an endoprosthesis can include a biocompatible material capable of expansion upon exposure to the environment within the body lumen. Examples of such biocompatible materials can include a suitable hydrogel, hydrophilic polymer, biodegradable polymers, bioabsorbable polymers. Examples of such polymers can include poly(alpha-hydroxy esters), polylactic acids, polylactides, poly-L-lactide, poly-DL-lactide, poly-L-lactide-co-DL-lactide, polyglycolic acids, polyglycolide, polylactic-co-glycolic acids, polyglycolide-co-lactide, polyglycolide-co-DL-lactide, polyglycolide-co-L-lactide, polyanhydrides, polyanhydride-co-imides, polyesters, polyorthoesters, polycaprolactones, polyesters, polyanydrides, polyphosphazenes, polyester amides, polyester urethanes, polycarbonates, polytrimethylene carbonates, polyglycolide-co-trimethylene carbonates, poly(PBA-carbonates), polyfumarates, polypropylene fumarate, poly(p-dioxanone), polyhydroxyalkanoates, polyamino acids, poly-L-tyrosines, poly(beta-hydroxybutyrate), polyhydroxybutyrate-hydroxyvaleric acids, combinations thereof, or the like. For example, a self-expandable endoprosthesis can be delivered to the desired location in an isolated state, and then exposed to the aqueous environment of the body lumen to facilitate expansion.

Furthermore, the endoprosthesis can be formed from a ceramic material. In one aspect, the ceramic can be a biocompatible ceramic which optionally can be porous. Examples of suitable ceramic materials include hydroxylapatite, mullite, crystalline oxides, non-crystalline oxides, carbides, nitrides, silicides, borides, phosphides, sulfides, tellurides, selenides, aluminum oxide, silicon oxide, titanium oxide, zirconium oxide, alumina-zirconia, silicon carbide, titanium carbide, titanium boride, aluminum nitride, silicon nitride, ferrites, iron sulfide, and the like. Optionally, the ceramic can be provided as sinterable particles that are sintered into the shape of an endoprosthesis or layer thereof.

Moreover, the endoprosthesis can include a radiopaque material to increase visibility during placement. Optionally, the radiopaque material can be a layer or coating any portion of the endoprosthesis. The radiopaque materials can be platinum, tungsten, silver, stainless steel, gold, tantalum, bismuth, barium sulfate, or a similar material.

### A. Biodegradable Coating Layers

It is further contemplated that the external surface and/or internal surface of the endoprosthesis (e.g., exterior and luminal surfaces) can be coated with another material having a composition different from the primary endoprosthetic material. The use of a different material to coat the surfaces can be beneficial for imparting additional properties to the endoprosthesis, such as providing radiopaque characteristics, drug-reservoirs, and improved biocompatibility.

In one configuration, the external and/or internal surfaces of an endoprosthesis can be coated with a biocompatible material. Such coatings can include hydrogels, hydrophilic and/or hydrophobic compounds, and polypeptides, proteins or amino acids or the like. Specific examples can include polyethylene glycols, polyvinylpyrrolidone ("PVP"), polyvinylalcohol ("PVA"), parylene, heparin, phosphorylcholine, or the like. A preferred coating material can include phosphorylcholine, as disclosed in U.S. Patent Nos. US Patent 6,015,815 entitled "TETRAZOL-CONTAINING RAPAMYCIN ANALOGS WITH SHORTENED HALF-LIVES".

The coatings can also be provided on the endoprosthesis to facilitate the loading or delivery of beneficial agents or drugs, such as therapeutic agents, pharmaceuticals and radiation therapies. As such, the endoprosthetic material and/or holes can be filled and/or coated with a biodegradable material.

Accordingly, the biodegradable material can contain a drug or beneficial agent to improve the use of the endoprosthesis. Such drugs or beneficial agents can include antithrombotics, anticoagulants, antiplatelet agents, thrombolytics, antiproliferatives, anti-inflammatories, agents that inhibit hyperplasia, inhibitors of smooth muscle proliferation, antibiotics, growth factor inhibitors, or cell adhesion inhibitors, as well as antineoplastics, antimitotics, antifibrins, antioxidants, agents that promote endothelial cell recovery, antiallergic substances, radiopaque agents, viral vectors having beneficial genes, genes, siRNA, antisense compounds, oligionucleotides, cell permeation enhancers, and combinations thereof. Another example of a suitable beneficial agent is described in US Patent No. 6,015,815 and US Patent No. 6,329,386 entitled "Tetrazole-containing rapamycin analogs with shortened half-lives".

In one configuration, the external surfaces of an endoprosthesis can include a coating comprised of polytetrafluorethylene ("PTFE"), expanded PTFE ("ePTFE"), Dacron, woven materials, cut filaments, porous membranes, harvested vessels and/or arteries, or others such materials to form a stent graft prosthesis. Similarly, a medical device, such as a valve, a flow regulator or monitor device, can be used with the endoprosthesis, such that the endoprosthesis functions as an anchor for the medical device within the body lumen.

In one configuration, different external surfaces of an endoprosthesis, such as a low stress zone less susceptible to flexing, can be coated with functional layers of an imaging compound or radiopaque material. The radiopaque material can be applied as a layer at low stress zones of the endoprosthesis. Also, the radiopaque material can be encapsulated within a biocompatible or biodegradable polymer and used as a coating. For example, the suitable radiopaque material can be palladium platinum, tungsten, silver, stainless steel, gold, tantalum, bismuth, barium sulfate, or a similar material. The radiopaque material can be applied as layers on selected surfaces of the endoprosthesis using any of a variety of well-known techniques, including cladding, bonding, adhesion, fusion, deposition or the like.

### B. Degradable Inter-Connectors

In one configuration, the inter-connectors and/or other stent elements of the endoprosthesis can be degradable. For instance, all or a portion of the inter-connector can be degraded after deployment by being biodegradable or energy-degradable. More generally, one or more portions of the endoprosthesis can be degraded after development by being biodegradable or energy-degradable. The biodegradable portions can be prepared from any of the biodegradable materials described herein as well as others.

A portion of the endoprosthesis, such as, but not limited to, an inter-connector, can be energy degradable by responding to light, RF, vibrational, or ultrasonic energy. As such, application of light, RF, vibrational, or ultrasonic energy to the particular portion of endoprosthesis can cause it to break. Alternatively, the portion of the endoprosthesis can include a material that undergoes a physical transition under light, which can have a specific or range of wavelengths, so that it more easily breaks and degrades under RF, vibrational, or ultrasonic energy. The energy can be applied to the energy degradable material after deployment via a catheter, such as the delivery catheter that delivers the endoprosthesis. In any event, any other type of inter-connector between annular elements can be fabricated of such an energy degradable material.

For example, a portion of the endoprosthesis, such as an inter-connector, can be fabricated of a material that is flexible during deployment and becomes brittle in response to application of a selected wavelength or range of wavelengths of electromagnetic radiation. As such, the energy can be applied to the endoprosthesis after deployment. Ultrasonic energy, such as that used in ultrasounds, can then be used to degrade the degradable portion of the endoprosthesis. This can be beneficial for removing, for instance, the inter-connector after deployment, which may help reduce inflammation and provide increased flexibility to the endoprosthesis. Advantageously, this can be used with an endoprosthesis or endoprosthetic system that includes an embolic filter.

### C. Matrix With Crack-Inhibiting Features

In addition to the foregoing compositions, a crack-inhibiting feature can be included within the matrix of the endoprosthesis. Exemplary crack-inhibiting features can include holes, fibers, particles, and bodies having multiple layers, such as planar layers or concentric layers. As such, any of the foregoing compositions can be impregnated and/or encapsulated with a suitable fibrous or particulate material. Also, an endoprosthesis can be prepared to include a plurality of holes that extend through the endoprosthetic body. Moreover, the endoprosthetic body can have multiple layers separated by junctions or boundaries that inhibit crack propagation.

### V. Method of Making Endoprostheses

Various different manufacturing techniques are well known and may be used for fabrication of the segmented endoprosthesis of the present invention. For example, the endoprosthesis can be formed from a hollow tube using a known technique, such as laser cutting, EDM, milling, chemical etching, hydro-cutting, and the like. Also, the endoprosthesis can be prepared to include multiple layers or coatings deposited through a cladding process such as vapor deposition, electroplating, spraying, or similar processes. Also, various other processes can be used such as those described below and or others known to those skilled in the art in light of the teaching contained herein.

Optionally, the endoprosthesis can be fabricated from a sheet of suitable material, where the sheet is rolled or bent about a longitudinal axis into the desired tubular shape. Additionally, either before or after being rolled into a tube, the material can be shaped to include endoprosthetic elements by being shaped with well-known techniques such as laser-cutting, milling, etching or the like. If desired, the lateral edges of the structure can be joined together, such as by welding or bonding, to form a closed tubular structure, or the lateral edges can remain unattached to form a coiled, rolled sheet or open tubular structure. Such fabrication techniques are described in more detail below and known to those skilled in the art.

### A. Sintering

A method of making an endoprosthesis in accordance with the present invention can include sintering sinterable particles to provide a sintered article having the shape of the endoprosthesis. The sintering can be conducted in molds that are in the shape of an endoprosthesis.

In one configuration, the sintered body can be obtained from a molded green body prepared by molding a mixture of sinterable particles with or without a binder into the shape of an endoprosthesis or body intermediate. Sintering a molded green body that has the shape of an endoprosthesis can provide a sintered body that can function as an endoprosthesis with no or minimal further processing. Alternatively, after the green body has been formed in the mold and sintered into a hardened endoprosthesis, the process can include shaping the sintered body with a stream of energy and/or matter in order to obtain a desired shape. Thus, sintering a green body in a mold can result in an endoprosthesis that is either ready for use, or requires additional processing or finishing.

Additionally, the sintered body can be shaped into an endoprosthesis as described herein. Also, the endoprosthesis can be further processed after sintering and/or shaping such as by grinding, sanding, or the like to provide enhanced surface characteristics.

### B. Drawing Concentric Tubes

In one configuration, a multilayered endoprosthesis in accordance with the present invention can be prepared by a drawing process that draws two or more distinct concentric tubes into a single tube having two or more layers. Additionally, such a drawing process can combine multiple concentric tubes into a single multilayered tube. The drawing process can be configured to produce junctions separating adjacent layers or bonds that bond adjacent layers. As such, the sequentially-adjacent concentric tubes can be drawn together and progressively reduced in a cross-sectional profile until the desired size and residual clamping stress is attained.

Accordingly, a metallurgical bond can be prepared with elements of each sequentially-concentric tube diffusing together and bonding so as to form a strong metallurgical bond. Such a metallurgical bond can be achieved by applying significant pressure and heat to the tubes. As such, a metallurgical bond can form a diffusion layer at the interface between sequentially-adjacent concentric tubes (i.e., layers). The characteristics of these diffusion layers can be controlled by the proper heat treatment cycle. In part, this is because the heat treatment, temperature, and time of processing can control the rates of transfer of the diffusing elements that produce the diffusion layers. Also, the pressure at the interface between layers can be developed so as to result in the residual radial clamping stress in the tube after drawing.

In one example of this process, an outer tube of nitinol, a middle tube of tantalum, and an inner tube of Nitinol can be arranged to form the composite structure. The multilayered material can be produced to result in bonding between the layers so as to achieve a residual clamping stress of at least about 344kPa (50 p.s.i.). Accordingly, the annealing process can be performed within a limited range of time and temperatures. For example, the lower limit can be at least about 843°C (1550°F) for at least six minutes, and the upper limit can be less than about 1010°C (1850°F) for less than 15 minutes.

In another configuration, a metallic interleaf layer can be placed between separate tubes so as to bond the tubes together and form a multilayered material. The multiple tubes separated by the metallic interleaf layer can be drawn together and progressively reduced until the desired cross-sectional profile and residual clamping stress is attained, as described above. The drawn tubes can be heat-treated to form a diffusion bond between the separate layers. As such, the metallic interleaf layer can enhance the diffusion rate or type of diffusing atoms that are transported across a diffusion region between one layer and the interleaf layer.

In one configuration, a multilayered sheet can be prepared to have separate layers of different materials or the same material. For example, the multilayered sheet can have a top layer of nitinol, a middle layer of tantalum, and a bottom layer of Nitinol. The sheet can be prepared by metallurgically bonding the layers prior to a deep drawing process, which is well known in the art. During the deep drawing process, the sheet can be placed over a die and forced into the die, such as by a punch or the like. A tube having a closed end and a defined wall thickness can be formed in the die. This process can be repeated using a series of dies that have progressively decreasing diameters until a multilayered tube is formed having the desired diameter and wall thickness. For certain material combinations, intermediate heat treatments can be performed between the progressive drawing operations to form a multilayered material that is resistant to delaminating. Once a multilayered tube of desired thickness and dimensions has been formed, the closed end and the curved edges can be cut off. Then, the tube can be heat treated, as described above, until proper inter-metallic bonds are formed between the layers.

### C. Shaping

Accordingly, an endoprosthetic material can be shaped by various methods as described in more detail below. Such shaping techniques can utilize streams of energy and/or streams of matter in order to impart shapes into the endoprosthetic material. The streams of energy include photons, electromagnetic radiation, atomic, and sub-atomic materials, as described above. On the other hand, the streams of matter are considered to include materials larger than atomic scale particles, and can be microscopic or macroscopic in size. In any event, the shaping can be designed to direct a stream of energy or a stream of matter at the endoprosthetic material to form an endoprosthetic element and/or holes therein.

In one configuration, a stream of energy can cut, shape, and/or form a tube into an endoprostheses by generating heat at the site where the stream intersects the material, as is well known in the art. The thermal interaction can elevate the local temperature to a point, which can cut, melt, shape, and/or vaporize portions of the endoprosthetic material from the rest of the material.

Accordingly, one configuration of the stream-cutting apparatus can operate and shape the endoprosthetic material by thermal interactions. As such, any of the thermal processes described herein can be used for thermal-cutting. For example, such thermal interactions can arise from laser beam treatment, laser beam machining, electron beam machining, electrical discharge machining, ion beam machining, and plasma beam machining.

In one configuration, by knowing the thermal properties of the endoprosthetic material, precise energy requirements can be calculated so that the thermal beam provides the appropriate or minimum energy for melting and/or vaporizing the material without significantly melting undesirable portions of the material. For example, laser beams are a common form of a stream of energy that can be used to shape the endoprosthetic material. Additionally, there are instances where a laser is preferred over all other cutting techniques because of the nature of the resulting endoprosthesis as well as the characteristics of the endoprosthetic material.

In one configuration, an endoprosthesis may be manufactured as described herein using a femtosecond laser. A femtosecond laser may be desirable in producing an endoprosthesis in accordance with the multilayered composite structure described above because it produces a smaller heat influence zone ("HIZ") or heat affected zone (HAZ) compared to other lasers, or it can substantially eliminate the HIZ or HAZ. In comparison, cutting an endoprosthesis using known methods can result in the tubular material being melted away, and thereby forming the pattern in the tubular member. Such melting can result in embrittlement of some materials due to oxygen uptake into the HIZ.

In one configuration, electrical discharge machining is used to shape endoprosthetic material and/or form holes in the endoprosthetic material as desired. As such, electrical discharge machining be capable of cutting all types of conductive materials such as exotic metal including titanium, hastaloy, kovar, inconel, hard tool steels, carbides, and the like. In electrical discharge, the main interaction between the stream of energy and the endoprosthetic material is thermal, where heat is generated by producing electrical discharges. This can lead to the endoprosthetic material being removed by melting and evaporation. Some examples of electrical discharge machining include wire electron discharge machining, CNC-controlled electrical discharge machining, sinker electrical discharge machining, small hole discharge machining, and the like.

In another configuration, a charged particle beam can be used for shaping the endoprosthetic material, wherein electron beams and ion beams exemplify charged particle beams. A charged particle beam is a group of electrically-charged particles that have approximately the same kinetic energy and move in approximately the same direction. Usually, the kinetic energies are much higher than the thermal energies of similar particles at ordinary temperatures. The high kinetic energy and the directionality of these charged beams can be useful for cutting and shaping of the green bodies, as described herein. Additionally, there are some instances where electron beams or ion beams are preferred over other cutting techniques.

In one configuration, a stream of chemical matter can be used in order to shape or form holes in the endoprosthetic material. Chemical-jet milling, for example, provides selective and controlled material removal by jet and chemical action. As such, the process is similar to water-jet cutting, which is described in more detail below. In any event, chemical-jet milling can be useful for shaping various types of endoprosthetic materials, which provides intricate shaping capabilities.

In another configuration, electrochemical shaping can be based on a controlled electrochemical dissolution process similar to chemical-jet milling an endoprosthetic material. As such, the endoprosthetic material can be attached to an electrical source in order to allow an electrical current to assist in the shaping.

In one configuration, hydro-cutting or water-jet cutting can be used to shape an endoprosthetic material. Hydro-cutting is essentially a water-jet technology that uses the high force and high pressure of a stream of water directed at the endoprosthetic material in order to cut and shape the material as desired. Hydro-cutting can be preferred over some of the other stream-cutting technologies because it can be free of heat, flame, and chemical reactions, and can provide a precise cold shaping technique. Also, heated water with or without being doped with reactive chemicals can also be used. Hydro-cutting is particularly suitable for polymeric endoprostheses, but can be used for metal materials when combined with abrasive particles, as described below.

Additionally, hydro-cutting can be enhanced by the introduction of particulate materials into the water feed line. As such, some hydro-cutting techniques utilize garnet or other rigid and strong materials in order to apply an abrasive cutting force along with the force applied by the water itself. Also, the hydro-cutting process can be used with or without inclusion of such abrasives.

Additionally, one of the benefits of hydro-cutting is the ability to reutilize and recycle the spent water-jet material. As such, the endoprosthetic material can be easily separated from the spent water, thereby enabling the recycling and reuse of the water during the hydro-cutting process.

In one configuration, sandblasting, which fits into the regime of stream of matter cutting, can be used to shape an endoprosthetic material by projecting a high energy stream of sand particles at the material. Sandblasting cuts materials in a manner similar to hydro-cutting, especially when the water-jet is doped with abrasive particulates. Additionally, various other particulate streams other than sand can be used in the stream-cutting techniques and machinery.

### D. Additional Processing

An additional step of passivation can be performed during the manufacturing stage of the endoprosthesis in order to form a homogeneous oxide layer for corrosion-resistance. The passivation process may be performed prior to installation of the markers or it may be performed after installation of the radiopaque markers. Alternatively, multiple passivation processes may be performed, once prior to application of the markers, and again after insertion of the markers.

As originally shaped and/or fabricated, the endoprosthesis can correspond to its delivery configuration, to a deployed configuration, or to a configuration therebetween. The endoprosthesis can be fabricated with a configuration at least slightly larger than the delivery configuration. In this manner, the endoprosthesis can be crimped or otherwise compressed into its delivery configuration in a corresponding delivery device.

In another configuration, the endoprosthesis can be originally fabricated from a tube having a diameter corresponding to the deployed configuration. In this manner, the longitudinally-free portions of the annular elements (e.g., elbow or foot not at a connection location) and circumferentially-free portions (e.g., the toe and/or heel portion of the foot extensions) can be maintained within the general cylindrical shape (e.g., diameter) of the endoprosthesis when deployed, so as to avoid such portions from extending radially inward when in the deployed configuration. The endoprosthesis can be designed to match the target vessel in which the endoprosthesis is to be deployed. For example, a stent can be provided with an outer diameter in the deployed configuration ranging from about 1 mm for neurological vessels to about 25 mm for the aorta. Similarly, a stent can be provided with a length ranging from about 5 mm to about 200 mm. Variations of these dimensions will be understood in the art based upon the intended application or indication for the endoprosthesis.

Also, the geometry of each component of the endoprosthesis or endoprosthetic element, such as the width, thickness, length and shape of the strut elements, inter-connectors, crossbars, connectors, elbows, foot portions, ankle portions, toe portions, heel portions and the like can be selected to obtain predetermined expansion, flexibility, foreshortening, coverage scaffolding, and cross-sectional profile characteristics. For example, longer crossbars and/or connectors can promote greater radial expansion or scaffolding coverage. The phase difference or circumferential alignment between adjacent annular elements likewise can be altered to control coverage and flexibility. Similarly, the number and placement of connection locations and, if present, the connectors, between longitudinally-adjacent annular elements can be selected to obtained the desired flexibility of the endoprosthesis. The number of elbows and/or foot extensions between connection locations also can be varied to achieve desired performance characteristics.

The present invention may be configured in other specific forms without departing from its essential characteristics. The described embodiments are to be considered in all respects only as illustrative and not restrictive. The scope of the invention is, therefore, indicated by the appended claims rather than by the foregoing description.

## Claims

1. A segmented endoprosthesis comprising:
a first annular element (110a);
a second annular element (110b) adjacent to the first annular element (110a);
a shock absorbing inter-connector (150) coupled to the first annular element (110a) and second annular element (110b), the shock absorbing inter-connector (150) being comprised of at least two bending members (152,154) and each of the at least two bending members (152,154) being comprised of at least one bending point (160,164);
a first coupling (156) that couples the shock absorbing inter-connector (150) to the first annular element (110a); and
a second coupling (158) that couples the shock absorbing inter-connector (150) to the second annular element (110b), **characterised in that** the first coupling (156) couples a first end of each of the at least two bending members (152,154) to the first annular element (110a), and the second coupling (158) couples a second end of each of the at least two bending members (152,154) to the second annular element (110b) such that the shock absorbing inter-connector (150) reduces the forces transferred from the first annual element (110a) to the second annular element (110b) during placement within a body lumen.

2. An endoprosthesis as in claim 1, wherein each of the at least two bending members (152,154) is resiliently flexible and configured to expand or collapse an angle between the two bending members (152,154).

3. An endoprosthesis as in claim 1, wherein the at least two bending points (160,164), first coupling (156), and second coupling (158) cooperate to define a shock absorbing inter-connector (150) having a collapsed orientation and an expanded orientation.

4. An endoprosthesis as in claim 3, wherein the shock absorbing inter-connector (150) is in the shape of a diamond that has a longitudinally collapsed configuration during deployment of the endoprosthesis.

5. An endoprosthesis as in claim 3, wherein the shock absorbing inter-connector (150) is in the shape of a diamond that has a laterally expanded configuration after deployment of the endoprosthesis.

6. An endoprosthesis as in claim 1, further comprising a second flexibly-resilient shock absorbing inter-connector (150) coupled to the first annular element (110a) and the second annular element (110d), the first shock absorbing inter-connector (150) collapsing and the second shock absorbing inter-connector (150) expanding when the endoprosthesis bends in a first direction, and the second shock absorbing inter-connector (150) collapsing and the first shock absorbing inter-connector (150) expanding when the endoprosthesis bends in a second direction opposite to the first direction.

7. An endoprosthesis as in any one of claims 1-6, further comprising one or more additional annular elements (110) longitudinally coupled to the first and second annular elements (110a,110b) through shock absorbing inter-connectors (150).

8. An endoprosthesis as in any one of claims 1-7, wherein each of the annular elements (110a,110b) includes interconnected strut elements.

9. An endoprosthesis as in claim 8, wherein the strut elements include one or more of crossbars, elbows, foot extensions, ankles, toes, heels, or intersections.

10. An endoprosthesis as in any one of claims 1-9, wherein the endoprosthesis is prepared from a shape memory material.

11. An endoprosthesis as in any one of claims 1-10, further comprising a radiopaque layer or coating.

12. An endoprosthesis as in any one of claims 1-11, further comprising a biodegradable coating.

13. An endoprosthesis as in any one of claims 1-12, further comprising a drug.

## Patentansprüche

1. Segmentierte Endoprothese, die folgendes aufweist:
ein erstes ringförmiges Element (110a);
ein zweites ringförmiges Element (110b), das dem ersten ringförmigen Element (110a) benachbart ist;
ein stoßdämpfendes Bindeglied (150), das mit dem ersten ringförmigen Element (110a) und dem zweiten ringförmigen Element (110b) verbunden ist, wobei das stoßdämpfende Bindeglied (150) aus mindestens zwei Biegeelementen (152, 154) besteht und jedes der mindestens zwei Biegeelemente (152, 154) aus mindestens einem Biegepunkt (160, 164) besteht;
eine erste Kupplung (156), die das stoßdämpfende Bindeglied (150) mit dem ersten ringförmigen Element (110a) verbindet; und
eine zweite Kupplung (158), die das stoßdämpfende Bindeglied (150) mit dem zweiten ringförmigen Element (110b) verbindet, **dadurch gekennzeichnet, dass** die erste Kupplung (156) ein erstes Ende eines jeden der mindestens zwei Biegeelemente (152, 154) mit dem ersten ringförmigen Element (110a) verbindet, und die zweite Kupplung (158) ein zweites Ende eines jeden der mindestens zwei Biegeelemente (152, 154) mit dem zweiten ringförmigen Element (110b) verbindet, so dass das stoßdämpfende Bindeglied (150) die Kräfte, die von dem ersten ringförmigen Element (110a) auf das zweite ringförmige Element (110b) während einer Platzierung innerhalb eines Körperlumens übertragen werden, reduziert.

2. Endoprothese nach Anspruch 1, wobei jedes der mindestens zwei Biegeelemente (152, 154) elastisch flexibel und zur Ausdehnung bzw. Vergrößerung oder zum Zusammenklappen bzw. Verkleinern eines Winkels zwischen den beiden Biegeelementen (152, 154) ausgelegt ist.

3. Endoprothese nach Anspruch 1, wobei die mindestens zwei Biegepunkte (160, 164), die erste Kupplung (156) und die zweite Kupplung (158) zur Definition eines stoßdämpfenden Bindeglieds (150) mit einer zusammengeklappten Ausrichtung und einer ausgedehnten Ausrichtung zusammenwirken.

4. Endoprothese nach Anspruch 3, wobei das stoßdämpfende Bindeglied (150) die Form eines Diamanten aufweist, der eine längsseitig zusammengeklappte Gestalt während des Einsatzes der Endoprothese aufweist.

5. Endoprothese nach Anspruch 3, wobei das stoßdämpfende Bindeglied (150) die Form eines Diamanten aufweist, der eine längsseitig ausgedehnte Gestalt nach dem Einsatz der Endoprothese aufweist.

6. Endoprothese nach Anspruch 1, die des Weiteren ein zweites flexibel-elastisches stoßdämpfendes Bindeglied (150) aufweist, das mit dem ersten ringförmigen Element (11 Oa) und dem zweiten ringförmigen Element (110d) verbunden ist, wobei das erste stoßdämpfende Bindeglied (150) zusammenklappt und das zweite stoßdämpfende Bindeglied (150) sich ausdehnt, wenn sich die Endoprothese in eine erste Richtung biegt, und wobei das zweite stoßdämpfende Bindeglied (150) zusammenklappt und das erste stoßdämpfende Bindeglied (150) sich ausdehnt, wenn sich die Endoprothese in eine zweite Richtung entgegengesetzt der ersten Richtung biegt.

7. Endoprothese nach einem der Ansprüche 1 bis 6, die des Weiteren ein oder mehrere zusätzliche ringförmige Elemente (110) aufweist, die längsseitig mit den ersten und zweiten ringförmigen Elementen (110a, 110b) durch stoßdämpfende Bindeglieder (150) verbunden sind.

8. Endoprothese nach einem der Ansprüche 1 bis 7, wobei jedes der ringförmigen Elemente (110a, 110b) miteinander verbundene Strebenelemente aufweist.

9. Endoprothese nach Anspruch 8, wobei die Strebenelemente eine oder mehrere der folgenden aufweisen: Querlatten, Winkelstücke, Schenkelverlängerungen, Knöchel, Fußstücke, Krängungen oder Schnittpunkte.

10. Endoprothese nach einem der Ansprüche 1 bis 9, wobei die Endoprothese aus einem Formgedächtniswerkstoff hergestellt ist.

11. Endoprothese nach einem der Ansprüche 1 bis 10, die des Weiteren eine radiopake Schicht oder Beschichtung aufweist.

12. Endoprothese nach einem der Ansprüche 1 bis 11, die des Weiteren eine biologisch abbaubare Beschichtung aufweist.

13. Endoprothese nach einem der Ansprüche 1 bis 12, die des Weiteren ein Arzneimittel aufweist.

## Revendications

1. Endoprothèse segmentée comprenant :
un premier élément annulaire (110a) ;
un deuxième élément annulaire (110b) situé de manière adjacente au premier élément annulaire (110a) ;
un interconnecteur absorbant les chocs (150) couplé au premier élément annulaire (110a) et au deuxième élément annulaire (110b), l'interconnecteur absorbant les chocs (150) étant constitué d'au moins deux éléments de flexion (152, 154) et chacun des au moins deux éléments de flexion (152, 154) étant constitué d'au moins un point de flexion (160, 164) ;
un premier élément de couplage (156) qui couple l'interconnecteur absorbant les chocs (150) au premier élément annulaire (110a) ; et
un deuxième élément de couplage (158) qui couple l'interconnecteur absorbant les chocs (150) au deuxième élément annulaire (110b), **caractérisé en ce que** le premier élément de couplage (156) couple une première extrémité de chacun des au moins deux éléments de flexion (152, 154) au premier élément annulaire (110a), et le deuxième élément de couplage (158) couple une deuxième extrémité de chacun des au moins deux éléments de flexion (152, 154) au deuxième élément annulaire (110b) de sorte que l'interconnecteur absorbant les chocs (150) réduise les forces transférées à partir du premier élément annulaire (110a) jusqu'au deuxième élément annulaire (110b) pendant le placement à l'intérieur d'une lumière corporelle.

2. Endoprothèse telle que revendiquée dans la revendication 1, dans laquelle chacun des au moins deux éléments de flexion (152, 154) est flexible de manière élastique et configuré pour élargir ou réduire un angle entre les deux éléments de flexion (152, 154).

3. Endoprothèse telle que revendiquée dans la revendication 1, dans laquelle les au moins deux points de flexion (160, 164), le premier élément de couplage (156) et le deuxième élément de couplage (158) coopèrent pour définir un interconnecteur absorbant les chocs (150) ayant une orientation repliée et une orientation étendue.

4. Endoprothèse telle que revendiquée dans la revendication 3, dans laquelle l'interconnecteur absorbant les chocs (150) est sous forme d'un losange qui a une configuration repliée longitudinalement lors du déploiement de l'endoprothèse.

5. Endoprothèse telle que revendiquée dans la revendication 3, dans laquelle l'interconnecteur absorbant les chocs (150) est sous forme d'un losange qui a une configuration étendue latéralement après le déploiement de l'endoprothèse.

6. Endoprothèse telle que revendiquée dans la revendication 1, comprenant en outre un deuxième interconnecteur absorbant les chocs élastique de manière flexible (150) couplé au premier élément annulaire (110a) et au deuxième élément annulaire (110d), le premier interconnecteur absorbant les chocs (150) se repliant et le deuxième interconnecteur absorbant les chocs (150) s'étendant lorsque l'endoprothèse se plie dans une première direction, et le deuxième interconnecteur absorbant les chocs (150) se repliant et le premier interconnecteur absorbant les chocs (150) s'étendant lorsque l'endoprothèse se plie dans une deuxième direction opposée à la première direction.

7. Endoprothèse telle que revendiquée dans l'une quelconque des revendications 1-6, comprenant en outre un élément ou plusieurs éléments annulaires supplémentaires (110) longitudinalement couplé(s) aux premier et deuxième éléments annulaires (110a, 110b) par le biais des interconnecteurs absorbant les chocs (150).

8. Endoprothèse telle que revendiquée dans l'une quelconque des revendications 1-7, dans laquelle chacun des éléments annulaires (110a, 110b) comporte des éléments d'entretoise interconnectés.

9. Endoprothèse telle que revendiquée dans la revendication 8, dans laquelle les éléments d'entretoise comportent un ou plusieurs éléments parmi des traverses, des coudes, des extensions formant embases, des chevilles, des orteils, des talons ou des intersections.

10. Endoprothèse telle que revendiquée dans l'une quelconque des revendications 1-9, dans laquelle l'endoprothèse est préparée à partir d'un matériau à mémoire de forme.

11. Endoprothèse telle que revendiquée dans l'une quelconque des revendications 1-10, comprenant en outre une couche ou un revêtement radio-opaque.

12. Endoprothèse telle que revendiquée dans l'une quelconque des revendications 1-11, comprenant en outre un revêtement biodégradable.

13. Endoprothèse telle que revendiquée dans l'une quelconque des revendications 1-12, comprenant en outre un médicament.
